(19) Europäisches Patentamt
European Patent Office
Office européen des brevets

(11) Publication number: **0 098 158**
**B1**

(12) # EUROPEAN PATENT SPECIFICATION

(45) Date of publication of patent specification: **19.11.87**

(21) Application number: **83303744.3**

(22) Date of filing: **29.06.83**

(51) Int. Cl.⁴: **C 07 C 127/22,**
C 07 C 157/12, A 01 N 47/34

(54) **Pesticidal 1-(alkyl-phenoxyaryl)-3-benzoyl ureas and process for preparation.**

(30) Priority: **30.06.82 US 393553**
**20.05.83 US 495331**

(43) Date of publication of application:
**11.01.84 Bulletin 84/02**

(45) Publication of the grant of the patent:
**19.11.87 Bulletin 87/47**

(84) Designated Contracting States:
**AT BE CH DE FR GB IT LI LU NL SE**

(56) References cited:
**EP-A-0 008 435**
**EP-A-0 050 321**
**FR-A-2 300 076**

**The file contains technical information submitted after the application was filed and not included in this specification**

(73) Proprietor: **UNION CARBIDE CORPORATION**
**39 Old Ridgebury Road**
**Danbury Connecticut 06817 (US)**

(72) Inventor: **Chou, David Teh-Wei**
**3101 Perrin Place**
**Raleigh North Carolina 27612 (US)**
Inventor: **Cain, Paul Alfred**
**1224 Willowbrook Drive**
**Gary North Carolina 27511 (US)**

(74) Representative: **Baverstock, Michael George Douglas et al**
**BOULT, WADE & TENNANT 27 Furnival Street**
**London, EC4A 1PQ (GB)**

**EP 0 098 158 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates in general to 1-(alkyl-phenoxyaryl)-3-benzoyl ureas and to a process for their preparation. In one aspect, this invention relates to benzoyl ureas which are useful as pesticides.

Prior to the present invention a few benzoyl ureas had been reported in the patent literature as having pesticidal activity. For example, US—A—3,992, 553 which issued on November 16, 1976, and US—A—4,041,177 which issued on August 9, 1977, both disclosed certain benzoylureido-diphenyl ethers which were indicated to possess insecticidal properties. Similarly, US—A—3,748,356 and 3,993,908 also disclosed certain substituted benzoyl ureas and stated that the compositions had strong insecticidal activity. US—A—4,148,902 which issued April 10, 1979 discloses substituted ((phenylamino)carbonyl) pyridine carboxamides and claims a method of controlling insects in addition to the compositions themselves. Additional disclosures of benzoyl ureas in the patent literature are found in US—A—4,166,124; 4,083,977; 4,160,834; 4,264,605; 4,064,267; and 4,005,223; 4,123,449; 4,068,002; 4,194,005; 4,275,077; 4,173,639; 3,989,842; Ger. Offen. 2,901,334 (EP 013-414); and DE 3,104,407 (EP 57-888).

Accordingly, one or more of the following objects can be achieved by the practice of this invention. It is an object of this invention to provide 1-(alkylphenoxyaryl)-3-benzoyl ureas. Another object of this invention is to provide certain 1-(alkylphenoxyphenyl)-3-benzoyl ureas which exhibit excellent insecticidal activity. A still further object of this invention is to provide benzoyl ureas, such as, 1-[2,4-dimethylphenoxy-3,6-dimethyl-5-chlorophenyl]-3-(2,6-difluorobenzoyl) urea, and

1-[4-(2,5-dimethyl-4-chlorophenoxy)-3,6-dimethyl-5-chlorophenyl]-3-(2,6-difluorobenzoyl)urea.

Another object is to provide processes for the preparation of these benzoyl ureas. A further object is to provide pesticidal compositions containing these benzoyl ureas as the active toxicant. Another object of the invention is to provide a method for controlling pests by the application of these pesticidal compositions. These and other objects will readily become apparent to those skilled in the art in the light of the teachings herein set forth.

In its broad aspect the invention relates to 1-(alkylphenoxyaryl)-3-benzoyl ureas, pesticidal compositions containing the same, and processes for their preparation and use. The benzoyl ureas of this invention can be represented by the following formula:

FORMULA 1

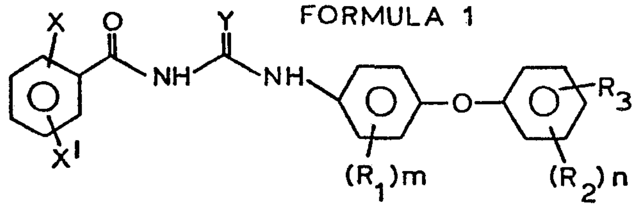

wherein

X, X' are independently hydrogen, halogen, $C_1$—$C_4$ alkyl, haloalkyl, polyhaloalkyl, alkoxy, polyhaloalkoxy,

Y represents oxygen or sulfur;

m, n are 1—4;

$R_1$ represents halogen, $C_1$—$C_4$ alkyl, haloalkyl, polyhaloalkyl, alkoxy,

$R_2$ represents halogen, $C_1$—$C_4$ alkyl, polyhaloalkyl, polyhaloalkoxy, $C_1$—$C_8$ alkylsulfonyl, $C_1$—$C_8$ alkoxy, $C_1$—$C_8$ alkylthio, $C_1$—$C_8$ dialkylamino, CN, $NO_2$, $CO_2R_4$, $CONHR_4$ wherein $R_4$ represents $C_1$—$C_8$ alkyl; and

$R_3$ represents $C_1$—$C_{12}$ alkyl, with the proviso that m may not be 0 or 1 when n is less than 2.

As indicated above, the invention relates 1-(alkyl-phenoxyaryl)-3-benzoyl ureas, pesticidal compositions containing the same, and processes for their preparation and use.

Preferred benzoyl urea compounds within the broad generic formula 1 are those having the formulae:

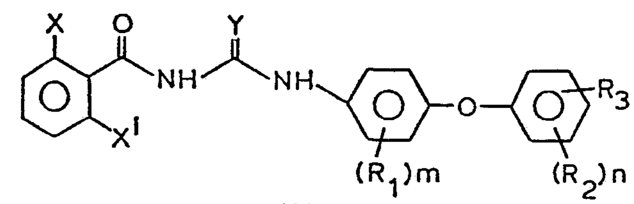

(2)

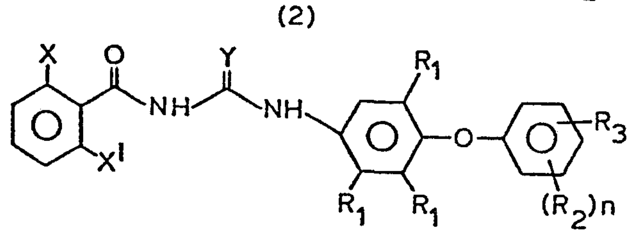

(3)

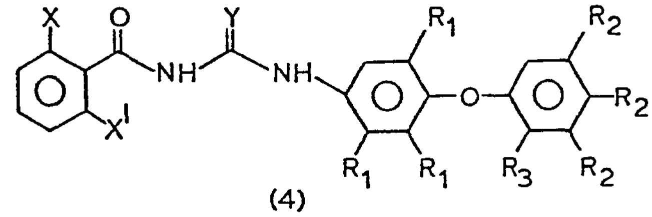

(4)

wherein X, $X^1$, Y, $R_1$, $R_2$, $R_3$, n and m are as indicated above.

Particularly preferred benzoyl ureas are those of the formulae:

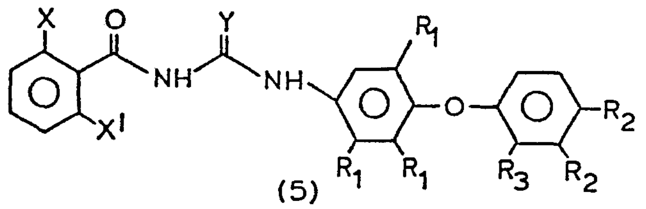

(5)

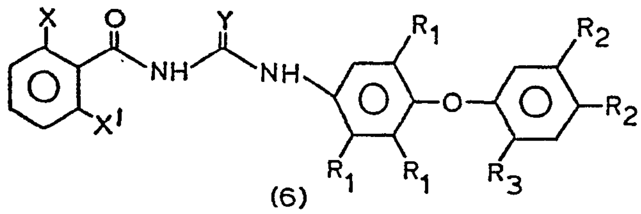

(6)

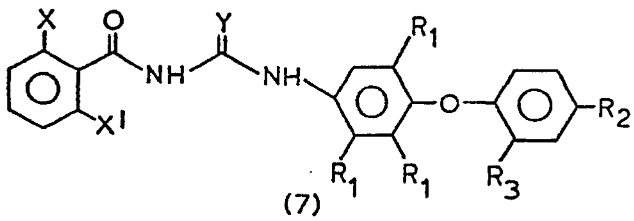

(7)

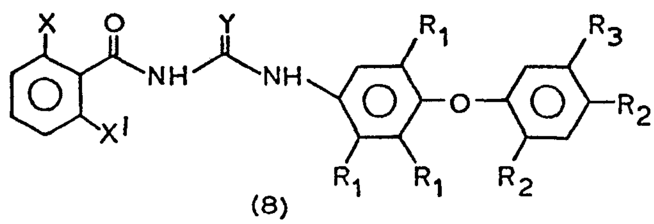

(8)

wherein X, X′, Y, $R_1$, $R_2$ and $R_3$ are as indicated above.

The following benzoyl urea compounds are illustrative of those encompassed by the above formulae and which can be prepared by the practice of this invention:

1-[4-(2-methyl-3,4-dichlorophenoxy)-3,6-dimethyl-5-chlorophenyl]-3-(2,6-difluorobenzoyl)urea,
1-[4-(2-methyl-3,4-dichlorophenoxy)-3,6-dimethyl-5-chlorophenyl]-3-(2-chlorobenzoyl)urea,
1-[4-(2-methylphenoxy)-3,6-dimethyl-5-chlorophenyl]-3-(2,6-difluorobenzoyl)urea,
11-[4-(2-methylphenoxy)-3,6-dimethyl-5-chlorophenyl]-3-(2,6-dichlorobenzoyl)urea,
1-[4-(2-methylphenoxy)-3,6-dimethyl-5-chlorophenyl]-3-(2-chlorobenzoyl)urea,
1-[4-(2-methylphenoxy)-3,6-dimethyl-5-chlorophenyl]-3-(2-chlorobenzoyl)thiourea,
1-[4-(2-methylphenoxy)-3,6-dimethyl-5-chlorophenyl]-3-(2-methylbenzoyl)thiourea,
1-[4-(2-methylphenoxy)-2,3,5,6-tetramethylphenyl]-3-(2,6-dimethylbenzoyl)urea,
1-[4-(2-methylphenoxy)-2,3,5,6-tetramethylphenyl]-3-(2,6-dimethylbenzoyl)thiourea,
1-[4-(2-methylphenoxy)-2,3,5,6-tetramethylphenyl]-3-(2,6-difluorobenzoyl)urea,
1-[4-(2-methylphenoxy)-2,3,5,6-tetramethylphenyl]-3-(2,6-dichlorobenzoyl)urea,
1-[4-(2-methylphenoxy)-2,3,5-trimethylphenyl]-3-(2,6-dichlorobenzoyl)urea,
1-[4-(2-methylphenoxy)-3,5-dichlorophenyl]-3-(2,6-difluorobenzoyl)urea,
1-[4-(2-methyl-4-bromophenoxy)-3,6-dimethyl-5-chlorophenyl]-3-(2,6-difluorobenzoyl)urea,

1-[4-(2-methyl-4-bromophenoxy)-3,6-dimethyl-5-chlorophenyl]-3-(2-chlorobenzoyl)urea,
1-[4-(2-methyl-4-bromophenoxy)-3,6-dimethyl-5-chlorophenyl]-3-(2-chlorobenzoyl)thiourea,
1-[4-(2-methyl-4-bromophenoxy)-3,6-dimethyl-5-chlorophenyl]-3-(2-methylbenzoyl)thiourea,
1-[4-(2-methyl-4-bromophenoxy)-2,3,5,6-tetramethylphenyl]-3-(2,6-difluorobenzoyl)urea,
1-[4-(2-methyl-4-bromophenoxy)-2,3,5-trimethylphenyl]-3-(2,6-difluorobenzoyl)urea,
1-[4-(2-methyl-4-bromophenoxy)-3,5-dimethylphenyl]-3-(2,6-difluorobenzoyl)urea,
1-[4-(2-methyl-4-bromophenoxy)-3,5-dichlorophenyl]-3-(2-chlorobenzoyl)urea,
1-[4-(2-bromo-4-methylphenoxy)-3,6-dimethyl-5-chlorophenyl]-3-(2,6-difluorobenzoyl)urea,
1-[4-(2-bromo-4-methylphenoxy)-3,6-dimethyl-5-chlorophenyl]-3-(2-chlorobenzoyl)urea,
1-[4-(2-bromo-4-methylphenoxy)-2,3,5,6-tetramethylphenyl]-3-(2-chlorobenzoyl)urea,
1-[4-(2-bromo-4-methylphenoxy)-3,5-dichlorophenyl]-3-(2,6-difluorobenzoyl)urea,
1-[4-(2-bromo-4-methylphenoxy)-3,5-dimethylphenyl]-3-(2-methylbenzoyl)thiourea,
1-[4-(2-methyl-4-t-butylphenoxy)-3,6-dimethyl-5-chlorophenyl]-3-(2,6-difluorobenzoyl)urea,
1-[4-(2-methyl-4-t-butylphenoxy)-3,6-dimethyl-5-chlorophenyl]-3-(2-chlorobenzoyl)urea,
1-[4-(2-methyl-4-t-butylphenoxy)-2,3,5,6-tetramethylphenyl]-3-(2,6-difluorobenzoyl)urea,
1-[4-(2-methyl-4-t-butylphenoxy)-3,5-dichlorophenyl]-3-(2-methylbenzoyl)thiourea,
1-[4-(2-methyl-4-chlorophenoxy)-3,6-dimethyl-5-chlorophenyl]-3-(2,6-difluorobenzoyl)urea,
1-[4-(2-methyl-4-chlorophenoxy)-3,5-dichlorophenyl]-3-(2-methylbenzoyl)thiourea,
1-[4-(2-methyl-4-chlorophenoxy)-2,3,5,6-tetramethylphenyl]-3-(2,6-difluorobenzoyl)urea,
1-[4-(2-methyl-4-chlorophenoxy)-3,6-dimethyl-5-chlorophenyl]-3-(2,6-dimethoxybenzoyl)urea,
1-[4-(4-nonylphenoxy)-3,6-dimethyl-5-chlorophenyl]-3-(2,6-difluorobenzoyl)urea,
1-[4-(4-nonylphenoxy)-2,3,5,6-tetramethylphenyl]-3-(2-chlorobenzoyl)thiourea,
1-[4-(4-nonylphenoxy)-3,5-dichlorophenyl]-3-(2,6-difluorobenzoyl)urea,
1-[4-(4-nonylphenoxy)-3,5-dimethylphenyl]-3-(2-methylbenzoyl)urea,
1-[4-(2-chloro-4-methylphenoxy)-3,6-dimethyl-5-chlorophenyl]-3-(2,6-difluorobenzoyl)urea,
1-[4-(2-chloro-4-methylphenoxy)-3,6-dimethyl-5-chlorophenyl]-3-(2-chlorobenzoyl)thiourea,
1-[4-(2-chloro-4-methylphenoxy)-3,5-dichlorophenyl]-3-(2,6-difluorobenzoyl)urea,
1-[4-(2-chloro-4-methylphenoxy)-2,3,5,6-tetramethylphenyl]-3-(2,6-difluorobenzoyl)urea,
1-[4-(3,4,5-trimethylphenoxy)-3,6-dimethyl-5-chlorophenyl]-3-(2,6-difluorobenzoyl)urea,
1-[4-(3,4,5-trimethylphenoxy)-3,6-dimethyl-5-chlorophenyl]-3-(2-chlorobenzoyl)thiourea,
1-[4-(3,4,5-trimethylphenoxy)-2,3,5,6-tetramethylphenyl]-3-(2-methylbenzoyl)thiourea,
1-[4-(3,4,5-trimethylphenoxy)-3,5-dichlorophenyl]-3-(2,6-difluorobenzoyl)urea,
1-[4-(2,3,5-trimethylphenoxy)-3,6-dimethyl-5-chlorophenyl]-3-(2,6-dimethoxybenzoyl)urea,
1-[4-(2,3,5-trimethylphenoxy)-2,3,5,6-tetramethylphenyl]-3-(2,6-difluorobenzoyl)urea,
1-[4-(2,3,5-trimethylphenoxy)-3-i-propylphenyl)-3-(2-methylbenzoyl)thiourea,
1-[4-(2,3,5-trimethylphenoxy)-3-trifluoromethylphenyl]-3-(2,6-difluorobenzoyl)urea,
1-[4-(2-methyl-4-trifluoromethylphenoxy)-3,6-dimethyl-5-chlorophenyl]-3-(2,6-difluorobenzoyl)urea,
1-[4-(2-methyl-4-trifluoromethylphenoxy)-3,6-dimethyl-5-chlorophenyl]-3-(2-chlorobenzoyl)thiourea,
1-[4-(2-methyl-4-trifluoromethylphenoxy)-3,5-dichlorophenyl]-3-(2,6-dimethoxybenzoyl)urea,
1-[4-(2-methyl-4-trifluoromethylphenoxy)-2,3,5-trimethylphenyl]-3-(2-methylbenzoyl)thiourea,
1-[4-(2,4-dimethylphenoxy)-2,3,6-trimethylphenyl]-3-(2-chlorobenzoyl)urea,
1-[4-(3,5-dimethylphenoxy)-2,6-dimethylphenyl]-3-(2,6-difluorobenzoyl)urea,
1-[4-(2-methyl-4-methoxyphenoxy)-3,6-dimethyl-5-chlorophenyl]-3-(2,6-difluorobenzoyl)urea,
1-[4-(2-methyl-4-dimethylaminophenoxy)-3,6-dimethyl-5-chlorophenyl]-3-(2,6-difluorobenzoyl)urea,
1-[4-(2-methyl-4-methylthiophenoxy)-3,6-dimethyl-5-chlorophenyl]-3-(2,6-difluorobenzoyl)urea,
1-[4-(2-methyl-4-methylsulfonylphenoxy)-3,6-dimethyl-5-chlorophenyl]-3-(2,6-difluorobenzoyl)urea,
1-[4-(2-methyl-4-dimethylaminophenoxy)-3,5-dimethylphenyl]-3-(2-methylbenzoyl)urea,
1-[4-(2-methyl-4-methoxyphenoxy)-3-chloro-6-methylphenyl]-3-(2-methylbenzoyl)urea,
1-[4-(2-methyl-4-methoxyphenoxy)-3,6-dimethyl-5-chlorophenyl]-3-(3,4-dichlorobenzoyl)urea,
1-[4-(2,3-dimethyl-4-bromophenoxy)-3,6-dimethyl-5-chlorophenyl]-3-(2-chlorobenzoyl)urea,
1-[4-(2,5-dimethyl-4-chlorophenoxy)-3,6-dimethyl-5-chlorophenyl]-3-(2,6-difluorobenzoyl)urea,
1-[4-(2,5-dimethylphenoxy)-3,6-dimethyl-5-chlorophenyl]-3-(2,6-difluorobenzoyl)urea,
1-[4-(2,5-dimethylphenoxy)-3,6-dimethyl-5-chlorophenyl]-3-(2-chlorobenzoyl)urea,
1-[4-(2,5-dimethyl-4-ethoxycarbonylphenoxy)-3,6-dimethyl-5-chlorophenyl]-3-(2,6-difluorobenzoyl)urea,
1-[4-(2,5-dimethyl-4-ethoxycarbonylphenoxy)-3,6-dimethyl-5-chlorophenyl]-3-(2-chlorobenzoyl)urea,
1-[4-(2-methyl-4-trifluoromethylphenoxy)-3,6-dimethyl-5-chlorophenyl]-3-(2-chlorobenzoyl)urea,
1-[4-(2,5-dimethyl-4-trifluoromethylphenoxy)-3,6-dimethyl-5-chlorophenyl]-3-(2,6-difluorobenzoyl)urea,
1-[4-(2,5-dimethyl-4-trifluoromethylphenoxy)-3,6-dimethyl-5-chlorophenyl]-3-(2-chlorobenzoyl)urea,
1-[4-(2,5-dimethyl-4-trifluoromethoxyphenoxy)-3,6-dimethyl-5-chlorophenyl]-3-(2,6-difluorobenzoyl)urea,
1-[4-(2,5-dimethyl-4-trifluoromethoxyphenoxy)-3,6-dimethyl-5-chlorophenyl]-3-(2-chlorobenzoyl)urea,
1-[4-(2,5-dimethyl-4-methoxyphenoxy)-3,6-dimethyl-5-chlorophenyl)-3-(2,6-diflurobenzoyl)urea,
1-[4-(2,5-dimethyl-4-methoxyphenoxy)-3,6-dimethyl-5-chlorophenyl]-3-(2-chlorobenzoyl)urea,

1-[4-(3,5-dimethyl-4-chlorophenoxy)-3,6-dimethyl-5-chlorophenyl]-3-(2,6-diflurobenzoyl)urea,
1-[4-(2,4,5-trimethylphenoxy)-3,6-dimethyl-5-chlorophenyl)-3-(2,6-diflurobenzoyl)urea,
1-[4-(2,4,5-trimethylphenoxy)-3,6-dimethyl-5-chlorophenyl)-3-(2-chlorobenzoyl)urea,
1-[4-(2,5-dimethyl-4-bromophenoxy)-3,6-dimethyl-5-chlorophenyl]-3-(2,6-difluorobenzoyl)urea,
1-[4-(2,5-dimethyl-4-cyanophenoxy)-3,6-dimethyl-5-chlorophenyl]-3-(2,6-diflurobenzoyl)urea,
1-[4-(2,5-dimethyl-4-cyanophenoxy)-3,6-dimethyl-5-chlorophenyl]-3-(2-chlorobenzoyl)urea,
1-[4-(2-methyl-4-cyanophenoxy)-3,6-dimethyl-5-chlorophenyl]-3-(2,6-diflurobenzoyl)urea,
1-[4-(2-methyl-4-cyanophenoxy)-3,6-dimethyl-5-chlorophenyl]-3-(2-chlorobenzoyl)urea,
1-[4-(2,4-dimethylphenoxy)-3,6-dimethyl-5-chlorophenyl]-3-(2,6-difluorobenzoyl)urea,
1-[4-(2,4-dimethylphenoxy)-3,6-dimethyl-5-chlorophenyl]-3-(2-chlorobenzoyl)urea,
1-[4-(2,5-dichloro-4-methylphenoxy)-3,6-dimethyl-5-chlorophenyl]-3-(2,6-difluorobenzoyl)urea,
1-[4-(2,5-dichloro-4-methylphenoxy)-3,6-dimethyl-5-chlorophenyl]-3-(2-chlorobenzoyl)urea,
1-[4-(2-methyl-4,5-dichlorophenoxy)-3,6-dimethyl-5-chlorophenyl]-3-(2,6-difluorobenzoyl)urea,
1-[4-(2-methyl-4,5-dichlorophenoxy)-3,6-dimethyl-5-chlorophenyl]-3-(2-chlorobenzoyl)urea, and
1-[4-(2,5-dimethyl-4-chlorophenoxy)-3-methylphenyl]-3-(2,6-difluorobenzoyl)urea.

The benzoyl ureas of this invention can be conveniently prepared by one or more methods. For example, the compounds of this invention may be prepared by reacting a alkylphenoxyaniline (9) with a benzoyl isocyanate or benzoyl isothiocyanate (10) according to Scheme I.

SCHEME I

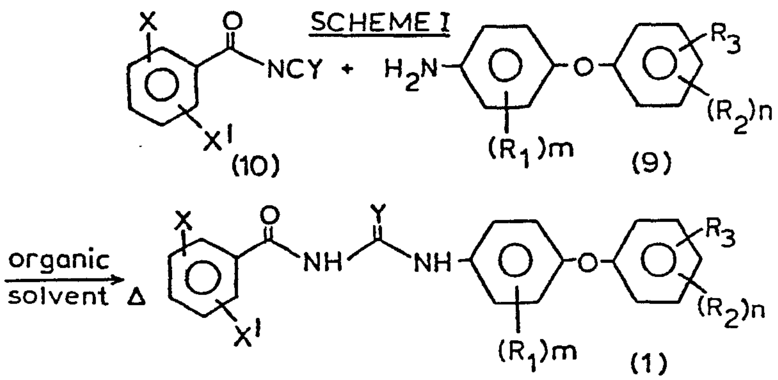


(10) (9) (1)

wherein X, X', Y, $R_1$, $R_2$, $R_3$, m, and n have the meaning given to Formula (1).

Alternatively, the compounds may be prepared by the reaction of an alkylphenoxyphenylisocyanate (12) with a benzamide (11) according to Scheme II.

SCHEME II

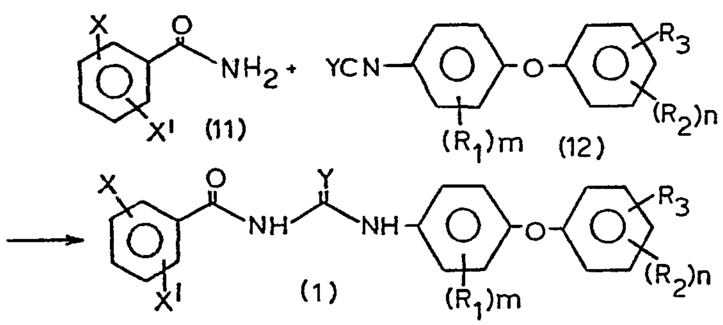

(11) (12) (1)

wherein X, X', Y, $R_1$, $R_2$, $R_3$, m, and n have the meaning given to Formula (1).

In general, the reactions illustrated in Schemes I and II can be carried out in organic solvents such as aromatic hydrocarbons or halogenated hydrocarbons. Solvents like toluene and 1,2-dichloroethane are preferred. These reactions proceed at temperatures ranging from room temperature to 150°C.

The intermediates shown in Schemes I and II can be prepared according to generally accepted procedures. Thus, the substituted benzoyl isocyanate (10) can be prepared from the corresponding benzamide following the general procedure of Speziale *et. al., J. Org. Chem.* 27, 3742 (1962).

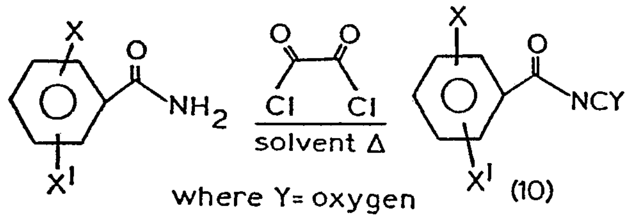

(10)

The substituted benzoyl isothiocyanate (10) can be prepared from the corresponding acid chloride with potassium thiocyanate. This procedure in general is similar to that employed by Ambelang, *et. al Jl. Amer. Chem. Soc.,* 61, 632 (1937).

The aniline (9) can be prepared according to Scheme III shown below.

SCHEME III

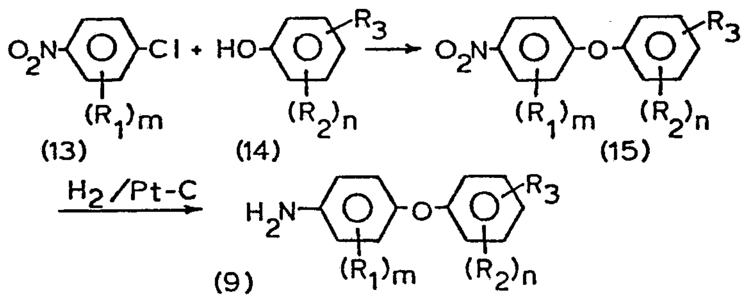

(13) (14) (15)

(9)

wherein $R_1$, $R_2$, $R_3$, m and n have the meaning given to Formula 1.

The reaction of substituted chloronitrobenzene (13) with substituted phenol (14) proceeds in the presence of base at elevated temperature to give the substituted nitroether (15). The reduction of nitroether (15) to aniline (9) can be achieved under hydrogen atmosphere using a heterogeneous hydrogenation catalyst. Suitable catalyst includes platinum or palladium on carbon or Raney nickel. The pressure ranging from 1,4 to 7 kp/cm$^2$ (20 to 100 PSI) at ambient temperature can be applied. Suitable solvents include aromatic hydrocarbon or alcohol. The reduction of nitroether (15) to aniline (9) can also be achieved by chemical method using the procedure of E. Enders, *et. al.,* GB—A—1,456,964.

The intermediates such as substituted chloronitrobenzene and substituted phenol are available commercially or may be prepared by well known method from chemical literature.

One particular chloronitrobenzene (16) was prepared by the route outlined in Scheme IV.

SCHEME IV

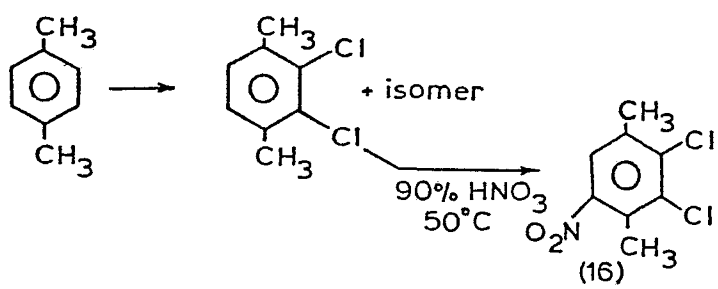

(16)

The aniline (9) can be converted to the isocyanate or isothiocyanate (12) by the reaction with phosgene or thiophosgene as shown below:

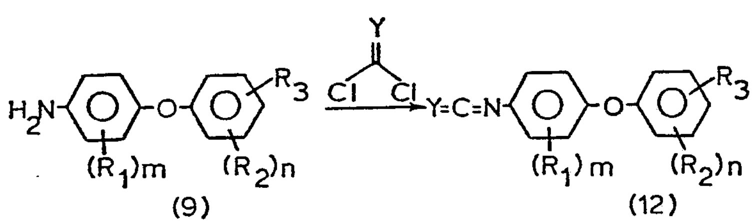
(9) (12)

wherein Y, $R_1$, $R_2$, $R_3$, m, and n have the meaning given to Formula 1.

The compounds contemplated in this invention may be employed as insecticides according to methods known to those skilled in the art. Pesticidal compositions containing the compounds as the active toxicant will usually comprise a carrier and/or diluent, either liquid or solid.

Suitable liquid diluents or carriers include water, petroleum distillates, or other liquid carriers with or without surface active agents. Liquid concentrates may be prepared by dissolving one of these compounds with a nonphytotoxic solvent such as acetone, xylene, nitrobenzene, cyclohexanone or dimethyl formamide and dispersing the toxicants in water with the aid of suitable surface active emulsifying and dispersing agents.

The choice of dispersing and emulsifying agents and the amount employed is dictated by the nature of the composition and the ability of the agent to facilitate the dispersion of the toxicant. Generally, it is desirable to use as little of the agent as is possible, consistent with the desired dispersion of the toxicant in the spray so that rain does not re-emulsify the toxicant after it is applied to the plant and wash it off the plant. Nonionic, anionic, or cationic dispersing and emulsifying agents may be employed, for example, the condensation products of alkylene oxides with phenol and organic acids, alkyl aryl sulfonates, complex ether alcohols, or quaternary ammonium compounds.

In the preparation of wettable powder or dust or granulated compositions, the active ingredient is dispersed in and on an appropriately divided solid carrier such as clay, talc, bentonite, diatomaceous earth, or fullers earth. In the formulation of the wettable powders the aforementioned dispersing agents as well as lignosulfonates can be included.

The required amount of the toxicants contemplated herein may be applied per 4047 $m^2$ (acre) treated in from 3.75 to 757 l (1 to 200 gallons) or more of liquid carrier and/or diluent or in from 2.13 to 227 kg (5 to 500 pounds) of inert solid carrier and/or diluent. The concentration in the liquid concentrate will usually vary from 10 to 95 percent by weight and in the solid formulations from 0.5 to 90 percent by weight. Satisfactory sprays, dusts, or granules for general use contain from 0.11 to 6.80 kg (1/4 to 15 pounds) of active toxicant per 4047 $m^2$ (acre).

The pesticides contemplated herein prevent attack by insects upon plants or other material to which the pesticides are applied, and they have relatively high residual toxicity. With respect to plants, they have a high margin of safety in that when used in sufficient amount to kill or repel the insects, they do not burn or injure the plant, and they resist weathering which includes wash-off caused by rain, decomposition by ultraviolet light, oxidation, or hydrolysis in the presence of moisture or, at least, such decomposition, oxidation, and hydrolysis as would materially decrease the desirable pesticidal characteristic of the toxicants or impart undesirable characteristics, for instance, phytotoxicity, to the toxicants. The toxicants are so chemically inert that they are now compatible with substantially any other constituents of the spray schedule, and they may be used in the soil, upon the seeds, or the roots of plants without injuring either the seeds or roots of plants. Mixtures of the active compounds may be employed if desired as well as combinations of the active compounds of this invention with other biologically active compounds or ingredients.

The following examples illustrate the best mode presently contemplated for the practice of the invention:

To a flask equipped with condenser, stirrer, under nitrogen, was added 3,6-dimethyl-4,5-dichloronitrobenzene (50 g, 0.23 mol), 2,4-dimethylphenol (38.5 g, 0.32 mol), potassium carbonate (50 g, 0.36 mol), and dimethylformamide (125 ml). The resulting mixture was heated at 90 ~ 100° for 72 hours. It was then cooled, filtered, and concentrated to give dark oil. It was partitioned between toluene (300 ml) and 4% sodium hydroxide (250 ml) and then separated. The organic layer was washed with water and brine, dried ($Na_2SO_4$) and concentrated to give dark oil. Solid was formed after the oil was triturated with hexane. The solid was washed with cold hexane and vacuum dried to give a tan powder (47.8 g, 0.16 mol); mp 78 ~ 80°.

Anal: $C_{16}H_{16}ClNO_3$
Calcd: C, 62.85; H, 5.27; N, 4.58
Found: C, 63.47; H, 5.35; N, 4.49

Example II

A. Preparation of 4-(2,4-dimethylphenoxy)-3,6-dimethyl-5-chloroaniline

To a solution of 4-(2,4-dimethylphenoxy)-3,6-dimethyl-5-chloronitrobenzene (25 g, 81.8 mmol) in toluene (250 ml) was added 5% platinum on carbon (1.0 g). The resulting mixture was subjected to hydrogenation at 1,4 kp/$cm^2$ (20 psi). After 4.5 hours, the reaction mixture was filtered through celite and concentrated to give an oil (24.5 g). It turned to a pinkish solid after high vacuum drying; mp 86 ~ 88°C.

Anal: $C_{16}H_{18}ClNO$
Calcd: C, 69.69; H, 6.53; N, 5.08
Found: C, 70.02; $H_3$ 6.60; N, 5.68

B. Preparation of 4-(3-methyl-4-methylthiophenoxy)-3,6-dimethyl-5-chloroaniline

To a solution of 4-(3-methyl-4-methylthiophenoxy)-3,6-dimethyl-5-chloronitrobenzene (5.0 g, 14.8 mmol) in 7.5 ml of p-dioxane was added $SnCl_2 \cdot 2H_2O$ (10.0 g, 44.4 mmol) and concentrated HCl (10.5 ml). The resulting mixture was heated up to reflex for 65 min., cooled, and poured into a mixture of NaOH (20 g), $H_2O$ (50 ml), and ice (50 g). The mixture was extracted twice with toluene. The combined organic extracts

were washed with 4% NaOH, $H_2O$, and brine. It was then dried ($Na_2SO_4$) and concentrated to yield a yellow oil. Solid formed after the oil was chilled and triturated with cold hexane and a trace of ethyl acetate. The solid was filtered and washed with hexane to give a beige powder (3.06 g, 9.9 mmol); mp 80—86°C (decomp.).

Anal: $C_{16}H_{18}ClNOS$
Calcd: C, 62.34; H, 5.89; N, 4.55
Found: C, 62.38; H, 5.89; N, 4.25

Example III

Preparation of 1-(2-Chlorobenzoyl)-3-[4-(2,4-dimethylphenoxy)-3,6-dimethyl-5-chlorophenyl]urea

To a warm solution of 4.94 g (17.9 mmol) of 4-(2,4-dimethylphenoxy)-3,6-dimethyl-5-chloroaniline in 12.5 ml of toluene was added 4.21 g of 2-chlorobenzoyl isocyanate in 1.5 ml of toluene. The resulting solution was heated at 90° for 0.5 hour. It was then cooled and diluted with 5 ml of hexane. The mixture was filtered and the solid was washed with 50% hexane in toluene. A white solid (6.32 g, 13.8 mmol) was obtained after vacuum dried at 80° overnight; mp 163—165°C.

Anal: $C_{24}H_{22}Cl_2N_2O_3$
Calcd: C, 63.03; H, 4.85; N, 6.12
Found: C, 63.33; H, 4.94; N, 6.09

Examples 1—164

In a manner similar to that employed in the preceding examples, and using one of the synthesis schemes previously disclosed, other urea compounds were prepared. The identity of the substituents on the generic formula and the analytical data are set forth in table I below:

TABLE I
1-(Alkylphenoxyaryl)-3-Benzoyl Ureas

| Example | Molecular Formula | XX′ | Y | $R_1$ | $R_2$ | $R_3$ | Calculated C | Calculated H | Calculated N | Found C | Found H | Found N |
|---|---|---|---|---|---|---|---|---|---|---|---|---|
| 1 | $C_{22}H_{16}Cl_2F_2N_2O_3$ | 2,6-$F_2$ | O | 3,5-Cl | 2-$CH_3$ | 4-$CH_3$ | 56.79 | 3.47 | 6.02 | 56.61 | 3.35 | 6.08 |
| 2 | $C_{22}H_{17}Cl_3N_2O_3$ | 2-Cl | O | 3,5-Cl | 2-$CH_3$ | 4-$CH_3$ | 56.97 | 3.70 | 6.04 | 56.84 | 3.61 | 6.02 |
| 3 | $C_{22}H_{16}Cl_4N_2O_3$ | 2,6-$Cl_2$ | O | 3,5-Cl | 2-$CH_3$ | 4-$CH_3$ | 53.03 | 3.24 | 5.62 | 52.38 | 3.15 | 5.72 |
| 4 | $C_{24}H_{22}F_2N_2O_3$ | 2,6-$F_2$ | O | 3,5-$(CH_3)_2$ | 2-$CH_3$ | 4-$CH_3$ | 67.91 | 5.23 | 6.60 | 67.23 | 5.10 | 6.62 |
| 5 | $C_{24}H_{22}ClFN_2O_3$ | 2,6-ClF | O | 3,5$(CH_3)_2$ | 2-$CH_3$ | 4-$CH_3$ | 65.35 | 5.03 | 6.35 | 65.22 | 5.03 | 6.29 |
| 6 | $C_{24}H_{23}ClN_2O_3$ | 2-Cl | O | 3,5$(CH_3)_2$ | 2-$CH_3$ | 4-$CH_3$ | 68.16 | 5.47 | 6.63 | 68.12 | 5.58 | 6.48 |
| 7 | $C_{24}H_{22}Cl_2N_2O_3$ | 2,6-$Cl_2$ | O | 3,5$(CH_3)_2$ | 2-$CH_3$ | 4-$CH_3$ | 63.02 | 4.85 | 6.13 | 58.72 | 4.16 | 6.33 |
| 8 | $C_{24}H_{22}Cl_2N_2O_3$ | 2-Cl | O | 3,5,6-$CH_3ClCH_3$ | 2-$CH_3$ | 4-$CH_3$ | 63.03 | 4.85 | 6.12 | 63.33 | 4.94 | 6.09 |
| 9 | $C_{24}H_{21}Cl_3N_2O_3$ | 2,6-$Cl_2$ | O | 3,5,6-$CH_3ClCH_3$ | 2-$CH_3$ | 4-$CH_3$ | 58.61 | 4.30 | 5.70 | 58.28 | 4.21 | 5.64 |
| 10 | $C_{24}H_{21}Cl_2FN_2O_3$ | 2,6-ClF | O | 3,5,6-$CH_3ClCH_3$ | 2-$CH_3$ | 4-$CH_3$ | 60.64 | 4.45 | 5.89 | 60.79 | 4.42 | 5.82 |
| 11 | $C_{24}H_{21}ClF_3N_2O_3$ | 2,6-$F_2$ | O | 3,5,6-$CH_3ClCH_3$ | 2-$CH_3$ | 4-$CH_3$ | 62.82 | 4.61 | 6.10 | 62.94 | 4.88 | 6.03 |
| 12 | $C_{22}H_{15}N_2O_3$ | 2-Cl | O | 3,5-$Cl_2$ | 2,3,4-$CH_3ClCl$ | 5-$CH_3$ | 49.61 | 2.84 | 5.26 | 49.99 | 2.90 | 5.21 |
| 13 | $C_{22}H_{14}Cl_6N_2O_3$ | 2,6-$Cl_2$ | O | 3,5-$Cl_2$ | 2,3,4-$CH_3ClCl$ | 5-$CH_3$ | 46.60 | 2.49 | 4.94 | 46.74 | 2.53 | 4.94 |
| 14 | $C_{22}H_{14}Cl_5FN_2O_3$ | 2,6-ClF | O | 3,5-$Cl_2$ | 2,3,4-$CH_3ClCl$ | 5-$CH_3$ | 47.99 | 2.56 | 5.09 | 47.21 | 2.80 | 5.01 |
| 15 | $C_{22}H_{14}Cl_4F_2N_2O_3$ | 2,6-$F_2$ | O | 3,5-$Cl_2$ | 2,3,4-CHClCl | 5-$CH_3$ | 49.47 | 2.64 | 5.24 | 49.44 | 2.74 | 5.17 |
| 16 | $C_{24}H_{22}Cl_2N_2O_2S$ | 2-Cl | S | 3,5,6-$CH_3ClCH_3$ | 2-$CH_3$ | 4-$CH_3$ | 60.89 | 4.68 | 5.92 | 61.13 | 4.83 | 6.05 |
| 17 | $C_{24}H_{22}Cl_2N_3O_3$ | 2-Cl | O | 3,5,6-$CH_3ClCH_3$ | 3-$CH_3$ | 5-$CH_3$ | 63.03 | 4.85 | 6.12 | 62.64 | 4.73 | 6.40 |
| 18 | $C_{24}H_{22}Cl_2N_2O_2S$ | 2-Cl | S | 3,6-$(CH_3)_2$-5-Cl | 3-$CH_3$ | 5-$CH_3$ | 60.89 | 4.68 | 5.92 | 61.12 | 4.73 | 5.94 |
| 19 | $C_{24}H_{21}Cl_3N_2O_3$ | 2,6-$Cl_2$ | O | 3,6-$(CH_3)_2$-5-Cl | 3-$CH_3$ | 5-$CH_3$ | 58.61 | 4.30 | 5.70 | 58.41 | 4.26 | 5.71 |
| 20 | $C_{24}H_{21}ClF_2N_2O_3$ | 2,6-$F_2$ | O | 3,6-$(CH_3)_2$-5-Cl | 3-$CH_3$ | 5-$CH_3$ | 62.82 | 4.61 | 6.10 | 63.23 | 4.67 | 6.08 |

TABLE I (continued)
1-(Alkylphenoxyaryl)-3-Benzoyl Ureas

| Example | Molecular Formula | XX′ | Y | $R_1$ | $R_2$ | $R_3$ | Calculated | | | Found | | |
|---|---|---|---|---|---|---|---|---|---|---|---|---|
| | | | | | | | C | H | N | C | H | N |
| 20 | $C_{23}H_{19}BrCl_2N_2O_3$ | 2-Cl | O | 3,6-$(CH_3)_2$-5-Cl | 4-Br | 2-$CH_3$ | 52.90 | 3.67 | 5.36 | 53.18 | 3.68 | 5.47 |
| 21 | $C_{23}H_{19}BrClFN_2O_2S$ | 2-F | S | 3,6-$(CH_3)_2$-5-Cl | 4-Br | 2-$CH_3$ | 52.94 | 3.67 | 5.37 | 53.13 | 3.68 | 5.37 |
| 22 | $C_{23}H_{18}BrCl_3N_2O_2$ | 2,6-$Cl_2$ | O | 3,6-$(CH_3)_2$-5-Cl | 4-Br | 2-$CH_3$ | 49.62 | 3.26 | 5.03 | 49.60 | 3.29 | 4.99 |
| 23 | $C_{23}H_{19}BrCl_2N_2O_2S$ | 2-Cl | S | 3,6-$(CH_3)_2$-5-Cl | 4-Br | 2-$CH_3$ | 51.32 | 3.56 | 5.20 | 51.63 | 3.53 | 5.20 |
| 24 | $C_{23}H_{18}BrClF_2N_2O_3$ | 2,6-$F_2$ | O | 3,6-$(CH_3)_2$-5-Cl | 4-Br | 2-$CH_3$ | 52.75 | 3.46 | 5.35 | 52.27 | 3.40 | 5.43 |
| 25 | $C_{23}H_{19}BrCl_2N_2O_3$ | 2-Cl | O | 3,6-$(CH_3)_2$-5-Cl | 2-Br | 4-$CH_3$ | 52.90 | 3.67 | 5.36 | 52.43 | 3.68 | 5.31 |
| 26 | $C_{23}H_{18}BrCl_3N_2O_3$ | 2,6-$Cl_2$ | O | 3,6-$(CH_3)_2$-5-Cl | 2-Br | 4-$CH_3$ | 49.62 | 3.26 | 5.03 | 49.75 | 3.23 | 5.00 |
| 27 | $C_{23}H_{18}BrClF_2N_2O_3$ | 2,6-$F_2$ | O | 3,6-$(CH_3)_2$-5-Cl | 2-Br | 4-$CH_3$ | 52.75 | 3.46 | 5.35 | 52.97 | 3.49 | 5.31 |
| 28 | $C_{23}H_{19}BrCl_2N_2O_2S$ | 2-Cl | S | 3,6-$(CH_3)_2$-5-Cl | 2-Br | 4-$CH_3$ | 51.32 | 3.56 | 5.20 | 50.70 | 3.55 | 5.00 |
| 29 | $C_{23}H_{19}Cl_3N_2O_3$ | 2-Cl | O | 3,6-$(CH_3)_2$-5-Cl | 4-Cl | 2-$CH_3$ | 57.82 | 4.01 | 5.86 | 57.51 | 4.00 | 5.66 |
| 30 | $C_{23}H_{18}Cl_4N_2O_3$ | 2,6-$Cl_2$ | O | 3,6-$(CH_3)_2$-5-Cl | 4-Cl | 2-$CH_3$ | 59.93 | 3.54 | 5.49 | 54.22 | 3.55 | 5.55 |
| 31 | $C_{23}H_{18}Cl_2F_2N_2O_3$ | 2,6-$F_2$ | O | 3,6-$(CH_3)_2$-5-Cl | 4-Cl | 2-$CH_3$ | 57.64 | 3.78 | 5.84 | 57.50 | 3.84 | 5.81 |
| 32 | $C_{23}H_{19}Cl_3N_2O_2S$ | 2-Cl | S | 3,6-$(CH_3)_2$-5-Cl | 4-Cl | 2-$CH_3$ | 55.94 | 3.88 | 5.67 | 55.98 | 3.94 | 5.60 |

TABLE I (continued)
1-(Alkylphenoxyaryl)-3-Benzoyl Ureas

| Example | Molecular Formula | XX′ | Y | $R_1$ | $R_2$ | $R_3$ | Calculated C | Calculated H | Calculated N | Found C | Found H | Found N |
|---|---|---|---|---|---|---|---|---|---|---|---|---|
| 33 | $C_{23}H_{19}Cl_3N_2O_3$ | 2-Cl | O | 3,6-$(CH_3)_2$-5-Cl | 2-Cl | 4-$CH_3$ | 57.82 | 4.01 | 5.86 | 58.20 | 4.17 | 5.72 |
| 34 | $C_{23}H_{18}Cl_4N_2O_3$ | 2,6-$Cl_2$ | O | 3,6-$(CH_3)_2$-5-Cl | 2-Cl | 4-$CH_3$ | 53.93 | 3.54 | 5.49 | 53.91 | 3.51 | 5.30 |
| 35 | $C_{23}H_{18}Cl_2F_2N_2O_3$ | 2,6-$F_2$ | O | 3,6-$(CH_3)_2$-5-Cl | 2-Cl | 4-$CH_3$ | 57.64 | 3.78 | 5.84 | 57.82 | 3.84 | 5.73 |
| 36 | $C_{23}H_{19}Cl_3N_2O_2S$ | 2-Cl | S | 3,6-$(CH_3)_2$-5-Cl | 2-Cl | 4-$CH_3$ | 55.94 | 3.88 | 5.67 | 55.78 | 3.91 | 5.55 |
| 37 | $C_{25}H_{25}ClN_2O_2S$ | 2-$CH_3$ | S | 3,6-$(CH_3)_2$-5-Cl | 2-$CH_3$ | 4-$CH_3$ | 66.28 | 5.56 | 6.18 | 65.12 | 5.73 | 5.98 |
| 38 | $C_{24}H_{22}ClFN_2O_2S$ | 2-F | S | 3,6-$(CH_3)_2$-5-Cl | 2-$CH_3$ | 4-$CH_3$ | 63.08 | 4.85 | 6.13 | 62.91 | 4.83 | 6.14 |
| 39 | $C_{27}H_{28}Cl_2N_2O_3$ | 2-Cl | O | 3,6-$(CH_3)_2$-5-Cl | 2-$CH_3$ | 4-t-butyl | 64.93 | 5.65 | 5.61 | 64.85 | 5.71 | 5.56 |
| 40 | $C_{27}H_{27}ClF_2N_2O_3$ | 2,6-$F_2$ | O | 3,6-$(CH_3)_2$-5-Cl | 2-$CH_3$ | 4-t-butyl | 64.74 | 5.43 | 5.59 | 64.70 | 5.73 | 5.65 |
| 41 | $C_{27}H_{28}Cl_2N_2O_2S$ | 2-Cl | S | 3,6-$(CH_3)_2$-5-Cl | 2-$CH_3$ | 4-t-butyl | 62.91 | 5.48 | 5.43 | 62.96 | 5.58 | 5.38 |
| 42 | $C_{27}H_{28}ClFN_2O_2S$ | 2-F | S | 3,6-$(CH_3)_2$-5-Cl | 2-$CH_3$ | 4-t-butyl | 64.98 | 5.66 | 5.61 | 65.24 | 5.85 | 5.52 |
| 43 | $C_{25}H_{24}Cl_2N_2O_3$ | 2-Cl | O | 3,6-$(CH_3)_2$-5-Cl | 2,3-$(CH_3)_2$ | 5-$CH_3$ | 63.69 | 5.13 | 5.94 | 63.68 | 5.30 | 5.99 |
| 44 | $C_{25}H_{23}Cl_3N_2O_3$ | 2,6-$Cl_2$ | O | 3,6-$(CH_3)_2$-5-Cl | 2,3-$(CH_3)_2$ | 5-$CH_3$ | 59.36 | 4.58 | 5.54 | 59.52 | 4.82 | 5.47 |
| 45 | $C_{25}H_{23}Cl_2FN_2O_3$ | 2,6-ClF | O | 3,6-$(CH_3)_2$-5-Cl | 2,3-$(CH_3)_2$ | 5-$CH_3$ | 61.35 | 4.74 | 5.72 | 61.49 | 4.81 | 5.77 |
| 46 | $C_{25}H_{23}ClF_2N_2O_3$ | 2,6-$F_2$ | O | 3,6-$(CH_3)_2$-5-Cl | 2,3-$(CH_3)_2$ | 5-$CH_3$ | 63.49 | 4.90 | 5.92 | 63.58 | 5.20 | 5.92 |
| 47 | $C_{25}H_{24}Cl_2N_2O_2S$ | 2-Cl | S | 3,6-$(CH_3)_2$-5-Cl | 2,3-$(CH_3)_2$ | 5-$CH_3$ | 61.59 | 4.96 | 5.75 | 61.78 | 5.12 | 5.77 |
| 48 | $C_{25}H_{24}ClFN_2O_2S$ | 2-F | S | 3,6-$(CH_3)_2$-5-Cl | 2,3-$(CH_3)_2$ | 5-$CH_3$ | 63.74 | 5.14 | 5.95 | 63.85 | 5.22 | 5.94 |
| 49 | $C_{26}H_{27}ClN_2O_2S$ | 2-$CH_3$ | S | 3,6-$(CH_3)_2$-5-Cl | 2,3-$(CH_3)_2$ | 5-$CH_3$ | 66.86 | 5.83 | 6.00 | 67.35 | 5.86 | 5.91 |
| 50 | $C_{25}H_{24}Cl_2N_2O_3$ | 2-Cl | O | 3,6-$(CH_3)_2$-5-Cl | 3,4-$(CH_3)_2$ | 5-$CH_3$ | 63.69 | 5.13 | 5.94 | 63.68 | 5.21 | 5.87 |
| 51 | $C_{25}H_{23}Cl_3N_2O_3$ | 2,6-$Cl_2$ | O | 3,6-$(CH_3)_2$-5-Cl | 3,4-$(CH_3)_2$ | 5-$CH_3$ | 59.36 | 4.58 | 5.54 | 59.68 | 4.80 | 5.31 |

TABLE I (continued)
1-(Alkylphenoxyaryl)-3-Benzoyl Ureas

| Example | Molecular Formula | XX′ | Y | $R_1$ | $R_2$ | $R_3$ | Calculated C | Calculated H | Calculated N | Found C | Found H | Found N |
|---|---|---|---|---|---|---|---|---|---|---|---|---|
| 52 | $C_{25}H_{23}Cl_2FN_2O_3$ | 2,6-ClF | O | 3,6-$(CH_3)_2$-5-Cl | 3,4-$(CH_3)_2$ | 5-$CH_3$ | 61.35 | 4.74 | 5.72 | 61.60 | 4.84 | 5.79 |
| 53 | $C_{25}H_{23}ClF_2N_2O_3$ | 2,6-$F_2$ | O | 3,6-$(CH_3)_2$-5-Cl | 3,4-$(CH_3)_2$ | 5-$CH_3$ | 63.49 | 4.90 | 5.92 | 63.35 | 4.97 | 5.83 |
| 54 | $C_{25}H_{24}Cl_2N_2O_2S$ | 2-Cl | S | 3,6-$(CH_3)_2$-5-Cl | 3,4-$(CH_3)_2$ | 5-$CH_3$ | 61.59 | 4.96 | 5.75 | 61.65 | 5.05 | 5.70 |
| 55 | $C_{25}H_{24}ClFN_2O_2S$ | 2-F | S | 3,6-$(CH_3)_2$-5-Cl | 3,4-$(CH_3)_2$ | 5-$CH_3$ | 63.74 | 5.14 | 5.95 | 64.29 | 5.17 | 5.91 |
| 56 | $C_{26}H_{27}ClN_2O_2S$ | 2-$CH_3$ | S | 3,6-$(CH_3)_2$-5-Cl | 3,4-$(CH_3)_2$ | 5-$CH_3$ | 66.86 | 5.83 | 6.00 | 67.60 | 6.13 | 6.13 |
| 57 | $C_{25}H_{25}ClN_2O_3$ | 2-$CH_3$ | O | 3,6-$(CH_3)_2$-5-Cl | 2-$CH_3$ | 4-$CH_3$ | 68.72 | 5.77 | 6.41 | 67.84 | 5.65 | 6.30 |
| 58 | $C_{24}H_{12}ClFN_2O_3$ | 2-F | O | 3,6-$(CH_3)_2$-5-Cl | 2-$CH_3$ | 4-$CH_3$ | 65.38 | 5.03 | 6.35 | 65.25 | 5.13 | 6.21 |
| 59 | $C_{25}H_{26}N_2O_3$ | 2-$CH_3$ | O | 3,5-$(CH_3)_2$ | 2-$CH_3$ | 4-$CH_3$ | 74.60 | 6.51 | 6.96 | 74.81 | 6.66 | 6.88 |
| 60 | $C_{25}H_{26}N_2O_2S$ | 2-$CH_3$ | O | 3,5-$(CH_3)_2$ | 2-$CH_3$ | 4-$CH_3$ | 71.74 | 6.26 | 6.69 | 72.14 | 6.42 | 6.54 |
| 61 | $C_{24}H_{23}FN_2O_3$ | 2-F | O | 3,5-$(CH_3)_2$ | 2-$CH_3$ | 4-$CH_3$ | 70.92 | 5.70 | 6.89 | 71.09 | 5.70 | 6.98 |
| 62 | $C_{24}H_{22}Cl_2N_2O_4$ | 2-Cl | O | 3,6$(CH_3)_2$-5-Cl | 2-$OCH_3$ | 4-$CH_3$ | 60.90 | 4.68 | 5.92 | 60.85 | 4.57 | 5.84 |
| 63 | $C_{24}H_{21}Cl_3N_2O_4$ | 2,6-$Cl_2$ | O | 3,6$(CH_3)_2$-5-Cl | 2-$OCH_3$ | 4-$CH_3$ | 56.77 | 4.17 | 5.52 | 56.53 | 4.14 | 5.37 |
| 64 | $C_{24}H_{21}Cl_2FN_2O_4$ | 2,6-ClF | O | 3,6-$(CH_3)_2$ | 2-$OCH_3$ | 4-$CH_3$ | 58.67 | 4.31 | 5.70 | 59.07 | 4.47 | 5.63 |
| 65 | $C_{24}H_{21}ClF_2H_2O_4$ | 2,6-$F_2$ | O | 3,6-$(CH_3)_2$ | 2-$OCH_3$ | 4-$CH_3$ | 60.70 | 4.46 | 5.90 | 60.98 | 4.19 | 5.72 |
| 66 | $C_{25}H_{25}ClN_2O_4$ | 2-$CH_3$ | O | 3,6-$(CH_3)_2$ | 2-$OCH_3$ | 4-$CH_3$ | 66.29 | 5.56 | 6.18 | 66.57 | 5.56 | 6.29 |
| 67 | $C_{24}H_{22}ClFN_2O_3S$ | 2-F | S | 3,6-$(CH_3)_2$ | 2-$OCH_3$ | 4-$CH_3$ | 60.95 | 4.69 | 5.93 | 60.63 | 4.58 | 5.71 |
| 68 | $C_{24}H_{23}ClN_2O_2S$ | 2-Cl | S | 3,5-$(CH_3)_2$ | 2-$CH_3$ | 4-$CH_3$ | 65.76 | 5.28 | 6.38 | 66.09 | 5.50 | 6.27 |
| 69 | $C_{24}H_{22}Cl_2N_2O_3S$ | 2-Cl | O | 3,6-$(CH_3)_2$-5-Cl | 4-$SCH_3$ | 3-$CH_3$ | 58.90 | 5.43 | 5.72 | 58.63 | 4.58 | 5.68 |
| 70 | $C_{24}H_{21}ClF_2N_2O_3S$ | 2,6-$F_2$ | O | 3,6-$(CH_3)_2$-5-Cl | 4-$SCH_3$ | 3-$CH_3$ | 58.72 | 4.31 | 5.71 | 58.62 | 4.29 | 5.91 |
| 71 | $C_{26}H_{27}Cl_2N_3O_3$ | 2-Cl | O | 3,6-$(CH_3)_2$-5-Cl | 3-$CH_3$,4-$N(CH_3)_2$ | 5-$CH_3$ | 62.40 | 5.44 | 8.40 | 62.56 | 5.44 | 8.52 |

TABLE I (continued)
1-(Alkylphenoxyaryl)-3-Benzoyl Ureas

| Example | Molecular Formula | XX′ | Y | $R_1$ | $R_2$ | $R_3$ | Calculated C | Calculated H | Calculated N | Found C | Found H | Found N |
|---|---|---|---|---|---|---|---|---|---|---|---|---|
| 72 | $C_{26}H_{26}ClF_2N_3O_3$ | 2,6-$F_2$ | O | 3,6-$(CH_3)_2$-5-Cl | 3-$CH_3$,4-N$(CH_3)_2$ | 5-$CH^3$ | 62.22 | 5.22 | 8.37 | 62.81 | 4.93 | 8.20 |
| 73 | $C_{26}H_{26}Cl_3N_3O_3$ | 2,6-$Cl_2$ | O | 3,6-$(CH_3)_2$-5-Cl | 3-$CH_3$,4-N$(CH_3)_2$ | 5-$CH^3$ | 58.38 | 4.90 | 7.86 | 58.28 | 5.02 | 7.99 |
| 74 | $C_{26}H_{26}Cl_2FN_3O_3$ | 2,6-ClF | O | 3,6-$(CH_3)_2$-5-Cl | 3-$CH_3$,4-N$(CH_3)_2$ | 5-$CH^3$ | 60.24 | 5.06 | 8.10 | 60.15 | 5.13 | 7.77 |
| 75 | $C_{26}H_{27}ClFN_3O_3$ | 2-F | O | 3,6-$(CH_3)_2$-5-Cl | 3-$CH_3$,4-N$(CH_3)_2$ | 5-$CH^3$ | 64.52 | 5.62 | 8.68 | 64.57 | 5.63 | 8.64 |
| 76 | $C_{27}H_{30}ClN_3O_3$ | 2-$CH_3$ | O | 3,6-$(CH_3)_2$-5-Cl | 3-$CH_3$,4-N$(CH_3)_2$ | 5-$CH^3$ | 67.56 | 6.30 | 8.75 | 67.21 | 6.38 | 8.38 |
| 77 | $C_{23}H_{20}Cl_2N_2O_3$ | 2-Cl | O | 2-$CH_3$ 5-Cl | 2-$CH_3$ | 4-$CH_3$ | 62.31 | 4.55 | 6.32 | 62.65 | 4.92 | 6.27 |
| 78 | $C_{23}H_{19}ClF_2N_2O_3$ | 2,6-$F_2$ | O | 2-$CH_3$ 5-Cl | 2-$CH_3$ | 4-$CH_3$ | 62.10 | 4.31 | 6.30 | 62.45 | 4.78 | 6.30 |
| 79 | $C_{22}H_{17}BrCl_2N_2O_0$ | 2-Cl | O | 2-$CH_3$-5-Cl | 4-Br | 2-$CH_3$ | 51.99 | 3.37 | 5.51 | 52.51 | 3.49 | 5.56 |
| 80 | $C_{22}H_{16}BrClF_2N_2O_3$ | 2,6-$F_2$ | O | C-$CH_3$-5-Cl | 4-Br | 2-$CH_3$ | 51.84 | 3.16 | 5.50 | 51.70 | 3.32 | 5.37 |
| 81 | $C_{23}H_{20}BrClN_2O_3$ | 2-Cl | O | 3-$CH_3$ | 2-$CH_3$-4-Br | 6-$CH_3$ | 56.58 | 4.13 | 5.74 | 57.19 | 4.34 | 5.77 |
| 82 | $C_{23}H_{10}BrF_2N_2O_3$ | 2,6-$F_2$ | O | 3-$CH_3$ | 2-$CH_3$4-Br | 6-$CH_3$ | 56.41 | 3.91 | 5.72 | 56.96 | 4.14 | 5.66 |

TABLE I (continued)
1-(Alkylphenoxyaryl)-3-Benzoyl Ureas

| Example | Molecular Formula | XX′ | Y | $R_1$ | $R_2$ | $R_3$ | Calculated C | Calculated H | Calculated N | Found C | Found H | Found N |
|---|---|---|---|---|---|---|---|---|---|---|---|---|
| 83 | $C_{23}H_{20}Cl_2N_2O_3$ | 2-$CH_3$ | O | 2-$CH_3$-5-Cl | 4-Cl | 2-$CH_3$ | 62.31 | 4.55 | 6.32 | 62.36 | 4.51 | 6.38 |
| 84 | $C_{24}H_{23}ClN_2O_3$ | 2-$CH_3$ | O | 2-$CH_3$5-Cl | 4-$CH_3$ | 2-$CH_3$ | 68.16 | 5.48 | 6.62 | 68.37 | 5.55 | 6.53 |
| 85 | $C_{23}H_{20}ClFN_2O_3$ | 2-F | O | 2-$CH_3$5-Cl | 4-$CH_3$ | 2-$CH_3$ | 64.72 | 4.72 | 6.56 | 65.29 | 4.77 | 6.63 |
| 86 | $C_{22}H_{17}Cl_3FN_2O_3$ | 2-F | O | 2-$CH_3$5-Cl | 4-Cl | 2-$CH_3$ | 59.08 | 3.83 | 6.26 | 59.48 | 3.80 | 6.24 |
| 87 | $C_{23}H_{20}BrFN_2O_3$ | 2-F | O | 3-$CH_3$ | 4-Br-6-$CH_3$ | 2-$CH_3$ | 58.56 | 4.27 | 5.94 | 59.37 | 4.43 | 5.82 |
| 88 | $C_{24}H_{23}BrN_2O_3$ | 2-$CH_3$ | O | 3-$CH_3$ | 4-Br-6-$CH_3$ | 2-$CH_3$ | 61.62 | 4.96 | 5.99 | 62.83 | 5.22 | 5.85 |
| 89 | $C_{22}H_{17}BrClFN_2O_3$ | 2-F | O | 2-$CH_3$-5-Cl | 4-Br | 2-$CH_3$ | 53.74 | 3.48 | 5.70 | 53.75 | 3.59 | 5.43 |
| 90 | $C_{23}H_{20}BrClN_2O_3$ | 2-$CH_3$ | O | 2-$CH_3$-5-Cl | 4-Br | 2-$CH_3$ | 56.63 | 4.13 | 5.74 | 56.84 | 4.09 | 5.64 |
| 91 | $C_{23}H_{20}BrClN_2O_3$ | 2-Cl | O | 3,5-$(CH_3)_2$ | 4-Br | 2-$CH_3$ | 56.63 | 4.13 | 5.74 | 56.64 | 4.16 | 5.64 |
| 92 | $C_{23}H_{20}BrFN_2O_3$ | 2-F | O | 3,5-$(CH_3)_2$ | 4-Br | 2-$CH_3$ | 58.61 | 4.28 | 5.94 | 58.59 | 4.54 | 5.79 |
| 93 | $C_{23}H_{10}BrClN_2O_3$ | 2-Cl-6-F | O | 3,5-$(CH_3)_2$ | 4-Br | 2-$CH_3$ | 54.62 | 3.79 | 5.54 | 54.66 | 3.82 | 5.50 |
| 94 | $C_{23}H_{19}BrF_2N_2O_3$ | 2,6-$F_2$ | O | 3,5-$(CH_3)_2$ | 4-Br | 2-$CH_3$ | 56.46 | 3.91 | 5.72 | 56.61 | 4.30 | 5.61 |
| 95 | $C_{23}H_{19}BrClFN_2O_3$ | 2,6-ClF | O | 2,5-$(CH_3)_2$ | 4-Br | 2-$CH_3$ | 54.62 | 3.79 | 5.54 | 54.96 | 3.90 | 5.36 |
| 96 | $C_{23}H_{18}BrF_2N_2O_3$ | 2,6-$F_2$ | O | 2,5-$(CH_3)_2$ | 4-Br | 2-$CH_3$ | 56.46 | 3.91 | 5.72 | 56.76 | 4.07 | 5.59 |
| 97 | $C_{23}H_{20}BrClN_2O_3$ | 2-Cl | O | 2,5-$(CH_3)_2$ | 4-Br | 2-$CH_3$ | 56.63 | 4.13 | 5.74 | 56.64 | 4.20 | 5.64 |
| 98 | $C_{23}H_{20}Cl_2N_2O_3$ | 2-Cl | O | 2,5-$(CH_3)_2$ | 2-Cl | 4-$CH_3$ | 62.31 | 4.55 | 6.32 | 62.21 | 4.69 | 6.09 |
| 99 | $C_{23}H_{20}ClFN_2O_3$ | 2-F | O | 2-5-$(CH_3)_2$ | 2-Cl | 4-$CH_3$ | 64.72 | 4.55 | 6.32 | 64.93 | 4.82 | 6.54 |

TABLE I (continued)
1-(Alkylphenoxyaryl)-3-Benzoyl Ureas

| | | | | | | | Calculated | | | Found | | |
|---|---|---|---|---|---|---|---|---|---|---|---|---|
| Example | Molecular Formula | XX′ | Y | $R_1$ | $R_2$ | $R_3$ | C | H | N | C | H | N |
| 100 | $C_{24}H_{23}ClN_2O_3$ | 2-$CH_3$ | O | 2,5-$(CH_3)_2$ | 2-Cl | 4-$CH_3$ | 68.16 | 5.48 | 6.62 | 68.20 | 5.61 | 6.62 |
| 101 | $C_{24}H_{23}ClN_2O_3$ | $H_2$ | O | 3,6-$(CH_3)_2$-5-Cl | 2-$CH_3$ | 4-$CH_3$ | 68.16 | 5.48 | 6.62 | 68.01 | 5.78 | 6.42 |
| 102 | $C_{23}H_{20}BrClN_2O_3$ | $H_2$ | O | 3,6-$(CH_3)_2$-5-Cl | 4-Br | 2-$CH_3$ | 56.63 | 4.13 | 5.74 | 57.07 | 4.56 | 5.69 |
| 103 | $C_{24}H_{22}ClFN_2O_3$ | 2-F | O | 3,6-$(CH_3)_2$-5-Cl | 2-$CH_3$ | 5-$CH_3$ | 65.38 | 5.03 | 6.35 | 65.74 | 5.17 | 6.25 |
| 104 | $C_{24}H_{22}Cl_2N_2O_3$ | 2-Cl | O | 3,6-$(CH_3)_2$-5-Cl | 2-$CH_3$ | 5-$CH_3$ | 63.03 | 4.85 | 6.12 | 63.14 | 5.13 | 6.06 |
| 105 | $C_{24}H_{21}ClF_2N_2O_3$ | 26-$F_2$ | O | 3,6-$(CH_3)_2$-5-Cl | 2-$CH_3$ | 5-$CH_3$ | 62.86 | 4.62 | 6.11 | 62.90 | 4.67 | 6.01 |
| 106 | $C_{25}H_{25}ClN_2O_3$ | 2-$CH_3$ | O | 3,6-$(CH_3)_2$-5-Cl | 2-$CH_3$ | 5-$CH_3$ | 68.72 | 5.77 | 6.41 | 68.94 | 5.66 | 6.27 |
| 107 | $C_{26}H_{26}Cl_2N_2O_3$ | 2-Cl | O | 2-$CH_3$-5-Cl | 2-$CH_3$ | 4-t-butyl | 64.34 | 5.40 | 5.77 | 64.93 | 5.49 | 5.73 |
| 108 | $C_{26}H_{26}ClFN_2O_3$ | 2-F | O | 2-$CH_3$-5-Cl | 2-$CH_3$ | 4-t-butyl | 66.59 | 5.59 | 5.97 | 66.81 | 5.72 | 5.91 |
| 109 | $C_{26}H_{25}ClF_2N_2O_3$ | 2,6-$F_2$ | O | 2-$CH_3$-5-Cl | 2-$CH_3$ | 4-t-butyl | 64.13 | 5.18 | 5.75 | 64.36 | 5.24 | 5.72 |
| 110 | $C_{27}H_{29}ClN_2O_3$ | 2-$CH_3$ | O | 2-$CH_3$-5-Cl | 2-$CH_3$ | 4-t-butyl | 69.74 | 6.29 | 6.02 | 69.85 | 6.41 | 5.98 |
| 111 | $C_{24}H_{22}Cl_2N_2O_3$ | 2-Cl | O | 3,6-$(CH_3)_2$-5-Cl | 2-$CH_3$ | 3-$CH_3$ | 63.03 | 4.85 | 6.12 | 63.35 | 5.39 | 6.36 |
| 112 | $C_{24}H_{21}Cl_2FN_2O_3$ | 2-Cl,6-F | O | 3,6-$(CH_3)_2$-5-Cl | 2-$CH_3$ | 3-$CH_3$ | 60.64 | 4.45 | 5.89 | 61.29 | 4.46 | 5.78 |

TABLE I (continued)
1-(Alkylphenoxyaryl)-3-Benzoyl Ureas

| Example | Molecular Formula | XX′ | Y | $R_1$ | $R_2$ | $R_3$ | Calculated | | | Found | | |
|---|---|---|---|---|---|---|---|---|---|---|---|---|
| | | | | | | | C | H | N | C | H | N |
| 113 | $C_{24}H_{21}ClF_2N_2O_3$ | 2,6-$F_2$ | O | 3,6-$(CH_3)_2$-5-Cl | 2-$CH_3$ | 3-$CH_3$ | 62.82 | 4.61 | 6.10 | 63.04 | 4.89 | 6.21 |
| 114 | $C_{24}H_{21}Cl_3N_2O_3$ | 2-Cl | O | 3,6-$(CH_3)_2$-5-Cl | 2-$CH_3$-4-Cl | 3-$CH_3$ | 58.61 | 4.30 | 5.70 | 58.87 | 4.53 | 5.70 |
| 115 | $C_{24}H_{20}Cl_3FN_2O_3$ | 2-Cl,6-F | O | 3,6-$(CH_3)_2$-5-Cl | 2-$CH_3$-4-Cl | 3-$CH_3$ | 56.54 | 3.95 | 5.50 | 57.41 | 4.27 | 5.57 |
| 116 | $C_{24}H_{20}Cl_2F_2N_2O_3$ | 2,6-$F_2$O | O | 3,6-$(CH_3)_2$-5-Cl | 2-$CH_3$-4-Cl | 3-$CH_3$ | 58.43 | 4.09 | 5.68 | 58.59 | 4.27 | 5.65 |
| 117 | $C_{23}H_{20}Cl_2N_2O_3$ | 2-Cl | O | 3-$CH_3$ | 2-$CH_3$-4-Cl | 3-$CH_3$ | 62.31 | 4.55 | 6.32 | 62.63 | 4.62 | 6.38 |
| 118 | $C_{23}H_{19}Cl_2FN_2O_3$ | 2-Cl-6-F | O | 3-$CH_3$ | 2-$CH_3$-4-Cl | 3-$CH_3$ | 59.88 | 4.15 | 6.07 | 60.54 | 4.54 | 6.06 |
| 119 | $C_{23}H_{19}ClF_2N_2O_3$ | 2,6-$F_2$ | O | 3-$CH_3$ | 2-$CH_3$-4-Cl | 3-$CH_3$ | 62.10 | 4.30 | 6.30 | 62.55 | 4.62 | 6.27 |
| 120 | $C_{24}H_{21}Cl_3N_2O_3$ | 2-Cl | O | 3,6-$(CH_3)_2$-5-Cl | 2-$CH_3$-4-Cl | 5-$CH_3$ | 58.61 | 4.30 | 5.70 | 58.47 | 4.30 | 5.63 |
| 121 | $C_{24}H_{20}Cl_3FN_2O_3$ | 2-Cl,6-F | O | 3,6-$(CH_3)_2$-5-Cl | 2-$CH_3$-4-Cl | 5-$CH_3$ | 56.54 | 3.95 | 5.50 | 56.88 | 4.05 | 5.39 |
| 122 | $C_{24}H_{20}Cl_2F_2N_2O_3$ | 2,6-$F_2$ | O | 3,6-$(CH_3)_2$-5-Cl | 2-$CH_3$-4-Cl | 5-$CH_3$ | 58.43 | 4.09 | 5.68 | 58.72 | 4.18 | 5.68 |
| 123 | $C_{24}H_{21}Cl_3N_2O_3$ | 2-Cl | O | 3,6-$(CH_3)_2$-5-Cl | 3-$CH_3$-4-Cl | 5-$CH_3$ | 58.61 | 4.30 | 5.70 | 58.59 | 4.34 | 5.53 |
| 124 | $C_{24}H_{20}Cl_2F_2N_2O_3$ | 2,6-$F_2$ | O | 3,6-$(CH_3)_2$-5-Cl | 3-$CH_3$-4-Cl | 5-$CH_3$ | 58.43 | 4.09 | 5.68 | 58.38 | 4.17 | 5.53 |
| 125 | $C_{24}H_{21}BrCl_2N_2O_3$ | 2-Cl | O | 3,6-$(CH_3)_2$-5-Cl | 2-$CH_3$-4Br | 3-$CH_3$ | 53.75 | 3.95 | 5.22 | 53.72 | 4.03 | 5.53 |
| 126 | $C_{24}H_{20}BrCl_2F_2N_2O_3$ | 2,6-$F_2$ | O | 3,6-$(CH_3)_2$-5-Cl | 2-$CH_3$-4Br | 3-$CH_3$ | 53.60 | 3.75 | 5.21 | 53.27 | 3.78 | 5.07 |
| 127 | $C_{23}H_{20}BrCl_2N_2O_3$ | 2-Cl | O | 3-$CH_3$ | 2-$CH_3$-4Br | 3-$CH_3$ | 56.63 | 4.13 | 5.74 | 56.52 | 4.22 | 5.76 |
| 128 | $C_{23}H_{19}BrF_2N_2O_3$ | 2,6-$F_2$ | O | 3-$CH_3$ | 2-$CH_3$-4Br | 3-$CH_3$ | 56.46 | 3.91 | 5.72 | 56.65 | 4.09 | 5.94 |
| 129 | $C_{23}H_{20}Cl_2N_2O_3$ | 2-Cl | O | 3-$CH_3$ | 2-$CH_3$-4-Cl | 5-$CH_3$ | 62.31 | 4.55 | 6.32 | 62.17 | 4.65 | 6.30 |
| 130 | $C_{23}H_{19}Cl_2F_2N_2O_3$ | 2,6-$F_2$ | O | 3-$CH_3$ | 2-$CH_3$-4-Cl | 5-$CH_3$ | 62.10 | 4.30 | 6.30 | 61.95 | 4.42 | 6.19 |

TABLE I (continued)
1-(Alkylphenoxyaryl)-3-Benzoyl Ureas

| Example | Molecular Formula | XX′ | Y | $R_1$ | $R_2$ | $R_3$ | Calculated | | | Found | | |
|---|---|---|---|---|---|---|---|---|---|---|---|---|
| | | | | | | | C | H | N | C | H | N |
| 131 | $C_{24}H_{21}BrF_2N_2O_3$ | 2,6-$F_2$ | O | 3,5-$(CH_3)_2$ | 2-$CH_3$-4-Br | 3-$CH_3$ | m.p. 194 ~ 197°C | | | | | |
| 132 | $C_{26}H_{25}BrCl_2N_2O_3$ | 2-Cl | O | 3,6-$(CH_3)_2$-5-Cl | 4-Br | 2-s-butyl | 55.34 | 4.46 | 4.96 | 55.49 | 4.42 | 4.87 |
| 133 | $C_{26}H_{24}BrClF_2N_2O_3$ | 2,6-$F_2$ | O | 3,6-$(CH_3)_2$-5-Cl | 4-Br | 2-s-butyl | 55.19 | 4.28 | 4.95 | 55.78 | 4.28 | 4.81 |
| 134 | $C_{25}H_{22}ClF_3N_2O_3$ | 2-$CF_3$ | O | 3,6-$(CH_3)_2$-5-Cl | 2-$CH_3$ | 4-$CH_3$ | 61.17 | 4.52 | 5.71 | 61.45 | 4.66 | 5.63 |
| 135 | $C_{25}H_{24}Cl_2N_2O_3$ | 2-Cl | O | 3,6-$(CH_3)_2$-5-Cl | 2,4-$(CH_3)_2$ | 5-$CH_3$ | 63.70 | 5.13 | 5.94 | 63.64 | 5.24 | 6.03 |
| 136 | $C_{25}H_{23}ClF_2N_2O_3$ | 2,6-$F_2$ | O | 3,6-$(CH_3)_2$-5-Cl | 2,4-$(CH_3)_2$ | 5-$CH_3$ | 63.50 | 4.90 | 5.92 | 63.75 | 5.18 | 6.63 |
| 137 | $C_{24}H_{21}Cl_3N_2O_3$ | 2-Cl | O | 3,6-$(CH_3)_2$-5-Cl | 2-Cl,4-$CH_3$ | 5-$CH_3$ | 58.61 | 4.30 | 5.70 | 59.19 | 4.68 | 5.60 |
| 138 | $C_{24}H_{20}Cl_2F_2N_2O_3$ | 2,6-$F_2$ | O | 3,6-$(CH_3)_2$-5-Cl | 2-Cl,4-$CH_3$ | 5-$CH_3$ | 58.43 | 4.09 | 5.68 | 58.64 | 4.36 | 5.64 |
| 139 | $C_{23}H_{18}Cl_4N_2O_4$ | 2-Cl | O | 3,6-$(CH_3)_2$-5-Cl | 2,4-$Cl_2$ | 5-$CH_3$ | 53.93 | 3.54 | 5.47 | 54.16 | 3.79 | 5.41 |
| 140 | $C_{23}H_{17}Cl_3F_2N_2O_3$ | 2,6-$F_2$ | O | 3,6-$(CH_3)_2$-5-Cl | 2,4-$Cl_2$ | 5-$CH_3$ | 53.77 | 3.34 | 5.45 | 53.57 | 3.42 | 5.58 |
| 141 | $C_{22}H_{16}BrCl_3N_2O_3$ | 2-Cl | O | 3,5-$Cl_2$ | 2-$CH_3$,4-Br | 5-$CH_3$ | 48.69 | 2.97 | 5.16 | 49.39 | 3.01 | 5.07 |
| 142 | $C_{22}H_{15}BrCl_2F_2N_2O_3$ | 2,6-$F_2$ | O | 3,5-$Cl_2$ | 2-$CH_3$,4-Br | 5-$CH_3$ | 48.56 | 2.78 | 5.15 | 49.05 | 2.86 | 5.08 |
| 143 | $C_{24}H_{21}BrCl_2N_2O_3$ | 2-Cl | O | 3,6-$(CH_3)_2$-5-Cl | 2-$CH_3$,4-Br | 5-$CH_3$ | 53.75 | 3.76 | 5.22 | 53.96 | 4.04 | 5.20 |
| 144 | $C_{24}H_{20}BrClF_2N_2O_3$ | 2,6-$F_2$ | O | 3,6-$(CH_3)_2$-5-Cl | 2-$CH_3$,4-Br | 5-$CH_3$ | 53.60 | 3.75 | 5.21 | 53.69 | 3.78 | 5.16 |
| 145 | $C_{23}H_{20}Cl_2N_2O_2S$ | 2-Cl | S | 2-$CH_3$-5-Cl | 2-$CH_3$ | 4-$CH_3$ | 60.13 | 4.39 | 6.10 | 60.58 | 4.55 | 6.05 |
| 146 | $C_{22}H_{17}Cl_3N_2O_2S$ | 2-Cl | S | 2-$CH_3$-5-Cl | 4-Cl | 2-$CH_3$ | 55.07 | 3.57 | 5.84 | 55.86 | 3.61 | 5.64 |
| 147 | $C_{23}H_{20}Cl_2N_2O_2S$ | 2-$CH_3$ | S | 2-$CH_3$-5-Cl | 4-Cl | 2-$CH_3$ | 60.13 | 4.39 | 6.10 | 60.14 | 4.32 | 6.08 |
| 148 | $C_{24}H_{23}BrN_2O_2S$ | 2-$CH_3$ | S | 3-$CH_3$ | 2-$CH_3$,4-Br | 6-$CH_3$ | 59.58 | 4.79 | 5.79 | 60.48 | 5.04 | 5.71 |

TABLE I (continued)
1-(Alkylphenoxyaryl)-3-Benzoyl Ureas

| Example | Molecular Formula | XX′ | Y | $R_1$ | $R_2$ | $R_3$ | Calculated C | Calculated H | Calculated N | Found C | Found H | Found N |
|---|---|---|---|---|---|---|---|---|---|---|---|---|
| 149 | $C_{22}H_{17}BrCl_2N_2O_2S$ | 2-Cl | S | 2-$CH_3$,5-Cl | 4-Br | 2-$CH_3$ | 50.40 | 3.27 | 5.34 | 50.32 | 3.37 | 5.22 |
| 150 | $C_{23}H_{20}BrClN_2O_2S$ | 2-Cl | S | 3-$CH_3$ | 2-$CH_3$-4-Br | 6-$CH_3$ | 54.78 | 4.00 | 5.56 | 55.82 | 4.37 | 5.04 |
| 151 | $C_{24}H_{23}ClN_2O_2S$ | $H_2$ | S | 3,6-$(CH_3)_2$,5-Cl | 2-$CH_3$ | 4-$CH_3$ | 65.67 | 5.28 | 6.38 | 66.57 | 5.41 | 6.35 |
| 152 | $C_{23}H_{20}Cl_2N_2O_2S$ | $H_2$ | S | 3,6-$(CH_3)_2$,5-Cl | 4-Cl | 2-$CH_3$ | 60.13 | 4.39 | 6.10 | 60.39 | 4.69 | 6.13 |
| 153 | $C_{24}H_{21}Cl_3N_2O_2S$ | 2-Cl | S | 3,6-$(CH_3)_2$,5-Cl | 3-$CH_3$-4-Cl | 5-$CH_3$ | 56.76 | 4.17 | 5.52 | 56.96 | 4.25 | 5.57 |
| 154 | $C_{24}H_{21}BrCl_2N_2O_2S$ | 2-Cl | S | 3,6-$(CH_3)_2$,5-Cl | 2-$CH_3$-4-Br | 3-$CH_3$ | 52.19 | 3.83 | 5.07 | 52.61 | 3.92 | 4.91 |
| 155 | $C_{23}H_{20}BrClN_2O_2S$ | 2-Cl | S | 3-$CH_3$ | 2-$CH_3$-4-Br | 3-$CH_3$ | 54.83 | 4.00 | 5.56 | 54.98 | 4.18 | 5.57 |
| 156 | $C_{20}H_{20}Cl_2N_2O_2S$ | 2-Cl | S | 3-$CH_3$ | 2-$CH_3$-4-Cl | 5-$CH_3$ | 60.13 | 4.39 | 6.10 | 60.62 | 4.61 | 6.13 |
| 157 | $C_{26}H_{25}BrCl_2N_2O_2S$ | 2-Cl | S | 3,6-$(CH_3)_2$,5-Cl | 4-Br | 2-s-butyl | 53.81 | 4.34 | 4.83 | 54.16 | 4.36 | 5.20 |
| 158 | $C_{24}H_{21}ClF_2N_2O_2S$ | 2,6-$F_2$ | S | 3,6-$(CH_3)_2$,5-Cl | 2-$CH_3$ | 4-$CH_3$ | 60.69 | 4.46 | 5.90 | 60.35 | 4.57 | 5.98 |
| 159 | $C_{24}H_{19}Cl_2N_3O_3$ | 2-Cl | O | 3,6-$(CH_3)_2$,5-Cl | 4-CN | 2-$CH_3$ | 61.55 | 4.09 | 8.97 | 61.68 | 4.48 | 8.32 |
| 160 | $C_{24}H_{18}ClF_2N_3O_3$ | 2,6-$F_2$ | O | 3,6-$(CH_3)_2$,5-Cl | 4-CN | 2-$CH_3$ | 61.35 | 3.86 | 8.94 | 63.16 | 4.42 | 8.35 |
| 161 | $C_{25}H_{21}Cl_2N_3O_3$ | 2-Cl | O | 3,6-$(CH_3)_2$,5-Cl | 4-CN,5-$CH_3$ | 2-$CH_3$ | 62.25 | 4.39 | 8.71 | 62.21 | 4.59 | 8.50 |
| 162 | $C_{25}H_{20}ClF_2N_3O_3$ | 2,6-$F_2$ | O | 3,6-$(CH_3)_2$,5-Cl | 4-CN,5-$CH_3$ | 2-$CH_3$ | 62.06 | 4.17 | 8.68 | 61.72 | 4.46 | 8.49 |
| 163 | $C_{26}H_{23}ClF_2N_2O_5$ | 2,6-$F_2$ | O | 3,6-$(CH_3)_2$,5-Cl | 4-$CO_2C_2H_5$ | 2-$CH_3$ | m.p. 176 ~ 179°C | | | | | |
| 164 | $C_{26}H_{24}Cl_2N_2O_5$ | 2-Cl | O | 3,6-$(CH_3)_2$,5-Cl | 4-$CO_2C_2H_5$ | 2-$CH_3$ | m.p. 188 ~ 190°C | | | | | |

Certain representative examples of the compounds were evaluated to determine their pesticidal activity against certain insects, including a caterpillar and a beetle. The compounds were also tested for phytotoxicity on important economic crops including bean, soybean, corn, tomato and cotton. The compounds were further evaluated for mammalian toxicity.

Suspensions of the test compounds were prepared by dissolving one gram of compound in 50 milliliters of acetone in which had been dissolved 0.1 gram (10 percent of the weight of compound) of an alkylphenoxy polyethoxyethanol surfactant, as an emulsifying or dispersing agent. The resulting solution was mixed into 150 milliliters of water to give roughly 200 milliliters of a suspension containing the compound of finely divided form. The thus-prepared stock suspension contained 0.5 percent by weight of compound. The test concentrations in parts per million by weight employed in the tests described hereinbelow were obtained by appropriate dilutions of the stock suspension with water. The test procedures were as follows:

Southern Armyworm Leaf Spray Bait Test

Larvae of the southern armyworm (*Spodoptera eridania,* (Cram.)), reared on Tendergreen bean plants at a temperature of 53 ± 2.7°C (80° ± 5°F) and a relative humidity of 50 ± 5 percent, constituted the test insects.

The test compounds were formulated by diluting the stock suspension with water to give a suspension containing 500 parts of test compound per million parts of final formulation. Potted Tendergreen bean plants of standard height and age were placed on a revolving turntable and sprayed with 100—110 milliliters of test compound formulation by use of a DeVilbiss spray gun set at 40 psig air pressure. This application, which lasted 25 seconds, was sufficient to wet plants to run-off. As a control, 100—110 milliliters of a water-acetone-emulsifier solution containing no test compound were also sprayed on infested plants. When dry, the paired leaves were separated and each one was placed in a 9 centimeter Petri dish lined with moistened filter paper. Five randomly selected larvae were introduced into each dish and the dishes were closed. The closed dishes were labeled and held at 53—56°C (80°—85°F) for three days. Although the larvae could easily consume the whole leaf within twenty-four hours, no more food was added. Larvae which were unable to move the length of the body, even upon stimulation by prodding, were considered dead. Percent mortality was recorded for various concentration levels.

Mexican Bean Beetle Leaf Spray Test

Fourth instar larvae of the Mexican bean beetle (*Epilachna varivestis,* Muls.), reared on Tendergreen bean plants at a temperature of 53 ± 2.7°C (80° ± 5°F) and 50 ± 5 percent relative humidity, were the test insects.

The test compounds were formulated by diluting the stock suspension with water to give a suspension containing 500 parts of test compound per million parts of final formulation. Potted Tendergreen bean plants of standard height and age were placed on a revolving turntable and sprayed with 100—110 milliliters of test compound formulation by use of a DeVilbiss spray gun set at 40; psig air pressure. This application, which lasted 25 seconds, was sufficient to wet plants to run-off. As a control, 100—110 milliliters of a water-acetone-emulsifier solution containing no test compound were also sprayed on infested plants. When dry, the paired leaves were separated and each was placed in a 9 centimeter Petri dish lined with moistened filter paper. Five randomly selected larvae were introduced into each dish, and the dishes were closed. The closed dishes were labeled and held at a temperature of 80° ± 5°F, for three days. Although the larvae could easily consume the leaf within 24 to 48 hours, no more food was added. Larvae which were unable to move the length of the body, even upon stimulation, were considered dead.

Tobacco Budworm and Cotton Bollworm Leaf Spray Bait Test

Second instar larvae of the tobacco budworm (weighing about 4.5 mg) (*Heliothis virescens,* F.) and the cotton bollworm (weighing about 2.5 mg) (*Heliothis zea,* (Boddie)), obtained commercially and reared on artificial diet at a temperature of 53 ± 2.7°C (79° ± 5°F) and a relative humidity of 50 ± 5 percent, constituted the test insects.

Using a procedure similar to the above, but substituting cotton plants for snapbeans, treated and dried cotton leaves were introduced into 9 cm Petri dishes which were organized into groups of 10-dish sets. One randomly selected larvae was introduced into each dish of a ten dish set and the dishes were closed. The closed dishes were labelled and held at 53 ± 2.7°C (80° ± 5°F) for five days.

The biological properties of certain representative examples of the compounds of this invention are set forth in Table II below.

TABLE II
Biological Properties of Representative Benzoyl Ureas

| | Activity 500 ppm | |
|---|---|---|
| Example | SAW(1) | MBB(2) |
| 1 | A | A |
| 2 | A | A |
| 3 | A | A |
| 4 | A | A |
| 5 | A | A |
| 6 | A | A |
| 7 | A | A |
| 8 | A | A |
| 9 | A | A |
| 10 | A | A |
| 11 | A | A |
| 12 | A | A |
| 13 | A | A |
| 14 | A | A |
| 15 | A | A |
| 16 | A | A |
| 17 | A | A |
| 18 | A | A |
| 19 | A | A |
| 20 | A | A |
| 21 | A | A |
| 22 | A | A |
| 23 | A | A |
| 24 | A | A |
| 25 | A | A |
| 26 | A | A |

(1) Southern Armyworm
(2) Mexican Bean Beetle
(3) Code = A = Complete Control
B = Moderate Control
C = No control

TABLE II (Continued)
Biological Properties of Representative Benzoyl Ureas

| | Activity 500 ppm | |
|---|---|---|
| Example | SAW(1) | MBB(2) |
| 27 | A | A |
| 28 | A | A |
| 29 | A | A |
| 30 | A | A |
| 31 | A | A |
| 32 | A | A |
| 33 | A | A |
| 34 | A | A |
| 35 | A | A |
| 36 | A | A |
| 37 | A | A |
| 38 | A | A |
| 39 | A | A |
| 40 | A | A |
| 41 | A | A |
| 42 | A | A |
| 43 | A | A |
| 44 | A | A |
| 45 | A | A |
| 46 | A | A |
| 47 | A | A |
| 49 | A | A |
| 50 | C | A |
| 51 | C | A |
| 52 | A | A |
| 53 | A | A |

(1) Southern Armyworm
(2) Mexican Bean Beetle
(3) Code = A = Complete Control
B = Moderate Control
C = No control

TABLE II (Continued)
Biological Properties of Representative Benzoyl Ureas

| | Activity 500 ppm | |
|---|---|---|
| Example | SAW(1) | MBB(2) |
| 54 | C | A |
| 55 | C | A |
| 56 | C | A |
| 57 | A | A |
| 58 | A | A |
| 59 | A | A |
| 60 | A | A |
| 61 | A | A |
| 62 | A | A |
| 63 | A | A |
| 64 | A | A |
| 65 | A | A |
| 66 | A | A |
| 67 | A | A |
| 68 | A | A |
| 69 | A | A |
| 70 | A | A |
| 71 | C | A |
| 72 | A | A |
| 73 | C | A |
| 74 | A | A |
| 75 | A | A |
| 77 | A | A |
| 78 | A | C |
| 79 | A | A |
| 80 | A | A |

(1) Southern Armyworm
(2) Mexican Bean Beetle
(3) Code = A = Complete Control
B = Moderate Control
C = No control

TABLE II (Continued)
Biological Properties of Representative Benzoyl Ureas

| | Activity 500 ppm | |
|---|---|---|
| Example | SAW[1] | MBB[2] |
| 82 | B | B |
| 83 | A | A |
| 84 | A | A |
| 85 | A | A |
| 86 | A | A |
| 87 | A | B |
| 89 | A | A |
| 90 | A | A |
| 91 | A | A |
| 92 | A | A |
| 93 | A | A |
| 94 | A | A |
| 95 | A | A |
| 96 | A | A |
| 97 | A | A |
| 98 | A | A |
| 99 | A | A |
| 100 | A | A |
| 101 | A | C |
| 102 | A | A |
| 103 | A | C |
| 104 | A | A |
| 105 | A | A |
| 106 | A | A |
| 107 | A | A |
| 108 | A | A |

(1) Southern Armyworm
(2) Mexican Bean Beetle
(3) Code = A = Complete Control
B = Moderate Control
C = No control

TABLE II (Continued)
Biological Properties of Representative Benzoyl Ureas

| | Activity 500 ppm | |
|---|---|---|
| Example | SAW(1) | MBB(2) |
| 109 | A | A |
| 110 | A | A |
| 111 | A | A |
| 112 | A | A |
| 113 | A | A |
| 114 | A | A |
| 115 | A | A |
| 116 | A | A |
| 117 | A | A |
| 118 | A | A |
| 119 | A | A |
| 120 | A | A |
| 121 | A | A |
| 122 | A | A |
| 123 | A | A |
| 124 | A | A |
| 125 | A | A |
| 126 | A | A |
| 127 | A | A |
| 128 | A | A |
| 129 | A | A |
| 130 | A | A |
| 131 | A | A |
| 132 | A | A |
| 133 | A | A |
| 134 | A | A |
| 135 | A | A |

(1) Southern Armyworm
(2) Mexican Bean Beetle
(3) Code = A = Complete Control
B = Moderate Control
C = No control

TABLE II (Continued)
Biological Properties of Representative Benzoyl Ureas

| | Activity 500 ppm | |
|---|---|---|
| Example | SAW(1) | MBB(2) |
| 136 | A | A |
| 137 | A | A |
| 138 | A | A |
| 139 | A | A |
| 140 | A | A |
| 141 | A | A |
| 142 | A | A |
| 143 | A | A |
| 144 | A | A |
| 145 | A | A |
| 146 | A | A |
| 147 | A | A |
| 149 | A | A |
| 151 | A | A |
| 152 | A | A |
| 153 | C | A |
| 154 | A | A |
| 155 | A | A |
| 156 | A | A |
| 157 | A | C |
| 158 | A | A |
| 159 | A | A |
| 160 | A | A |

(1) Southern Armyworm
(2) Mexican Bean Beetle
(3) Code = A = Complete Control
B = Moderate Control
C = No control

Examples 165—170

In order to demonstrate the enhanced biological activity against the Southern Armyworm, representative benzoyl ureas were compared with known products. The results are set forth in Table III below:

TABLE III

Comparison of representative benzoyl ureas with closely related prior art compounds against Southern Armyworm

| Compound | Application rate (ppm) | Percent Control after 5 days |
|---|---|---|
| 1-(4-chlorophenyl)-3-(2,6-difluorobenzoyl)urea(1) | 10<br>5 | 100<br>40 |
| 1-[4-(4-methylphenylthio) phenyl]-3-(2,6-dichloro-benzoyl)urea(2) | 500<br>125<br>31 | 40<br>30<br>10 |
| 1-[4-(2,4-dimethylphenoxy)-3-chlorophenyl]-3-(2-chloro-benzoyl)urea(3) | 125<br>31<br>8 | 100<br>30<br>20 |
| 1-[4-(2,4-dimethylphenoxy)-3,6-dimethyl-5-chlorophenyl]-3-(2-chlorobenzoyl)urea (Example #8) | 2<br>1 | 100<br>100 |
| 1-[4-(2,4-dimethylphenoxy)-3,6-dimethyl-5-chlorophenyl] -3-(2-chlorobenzoyl)thiourea (Example #16) | 2<br>1 | 100<br>90 |
| 1-[4-(2,4-dimethylphenoxy) 3,5-dimethylphenyl]-3-(2,6-difluorobenzoyl)urea (Example #4) | 8<br>2 | 100<br>100 |

(1) Dimilin®, a known compound.
(2) a prior art compound. Ger. Offen. 2,901,334.
(3) a prior art compound. Ger. Offen. DE 3,104,407 (EP 57—888).

Examples 171—175

In order to demonstrate the enhanced biological activity against *Heliothis,* representative benzoyl ureas were compared with known products. The results are set forth in Table IV and V below:

TABLE IV

Comparison of representative benzoyl ureas with closely related prior art compounds against Heliothis zea

| Compound | $LD_{50}$ (ppm) Heliothis zea |
|---|---|
| 1-(4-chlorophenyl)-3-(2,6-difluorobenzoyl)urea(1) | 500 |
| 1-[4-(4-methylphenylthio)phenyl]-3-(2,6-dichlorobenzoyl)urea(2) | >100 |
| 1-[4-(2,4-dimethylphenoxy)-3-chlorophenyl]-3-(2,6-difluorobenzoyl)urea(3) | >100 |
| 1-[4-(2,4,5-trimethylphenoxy)-3,6-dimethyl-5-chlorophenyl]-3-(2,6-difluorobenzoyl)urea (Example #159) | 0.4 |
| 1-[4-(2,5-dimethyl-4-chlorophenoxy)-3,6-dimethyl-5-chlorophenyl]-3-(2,6-difluorobenzoyl)urea (Example #145) | 0.7 |

(1) Dimilin®, a known compound
(2) a prior art compound. Ger. Offen 2,901,334.
(3) a prior art compound. Ger. Offen. DE 3,104,407 (EP 57—888).

TABLE V

Comparison of representative benzoyl ureas with closely related prior art compounds against Heliothis virescens

| Compound | $LD_{50}$ (ppm) Heliothis virescens |
|---|---|
| 1-(4-chlorophenyl)-3-(2,6-difluorobenzoyl)urea(1) | 31 |
| 1-[4-(4-methylphenylthio)phenyl]-3-(2,6-dichlorobenzoyl)urea(2) | >100 |
| 1-[4-(2,4-dimethylphenoxy)-3-chlorophenyl]-3-(2,6-difluorobenzoyl)urea(3) | >100 |
| 1-[4-(2,5-dimethyl-4-chlorophenoxy)-3,6-dimethyl-5-chlorophenyl]-3-(2,6-difluorobenzoyl)urea (Example #145) | 0.63 |
| 1-[4-(2-methyl-4-t-butylphenoxy) 3,6-dimethyl-5-chlorophenyl]-3-(2,6-difluorobenzoyl)urea (Example #57) | 2.4 |

(1) Dimilin®, a known compound
(2) a prior art compound. Ger. Offen 2,901,334.
(3) a prior art compound. Ger. Offen. DE 3,104,407 (EP 57—888).

**Claims**

1. A 1-(alkyl-phenoxyaryl)-3-benzoyl urea of the general formula:

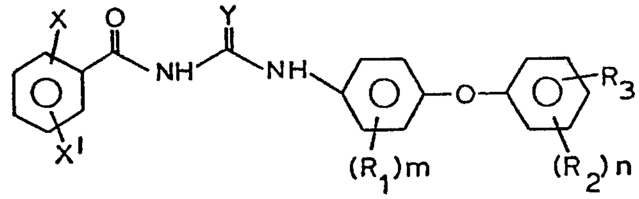

wherein

X, X′ are independently hydrogen, halogen, $C_1$—$C_4$ alkyl, haloalkyl, polyhaloalkyl, alkoxy, polyhaloalkoxy,

Y represents oxygen or sulfur;

m, n are independently 1—4;

$R_1$ represents halogen, $C_1$—$C_4$ alkyl, haloalkyl, polyhaloalkyl, or alkoxy,

$R_2$ represents halogen, $C_1$—$C_4$ alkyl, polyhaloalkyl, polyhaloalkoxy, $C_1$—$C_8$ alkylsulfonyl, $C_1$—$C_8$ alkoxy, $C_1$—$C_8$ alkylthio, $C_1$—$C_8$ dialkylamino, CN, $NO_2$, $CO_2R_4$, or $CONHR_4$ wherein $R_4$ represents $C_1$—$C_8$ alkyl; and

$R_3$ represents $C_1$—$C_{12}$ alkyl, with the proviso that m may not be 0 or 1 when n is less than 2.

2. A urea as claimed in claim 1 having the formula:

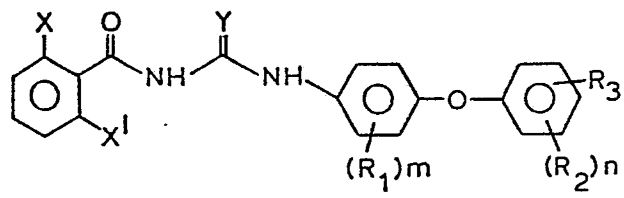

wherein X, X′, Y, $R_1$, $R_2$, $R_3$, m and n are as indicated in claim 1.

3. A urea as claimed in claim 1 having the formula:

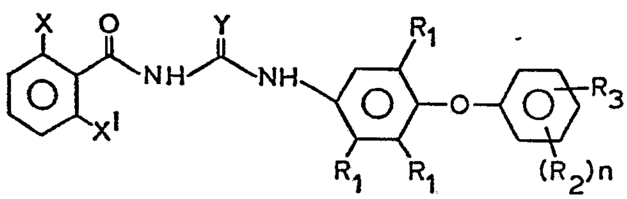

wherein X, X′, Y, $R_1$, $R_2$, $R_3$, m and n are as indicated in claim 1.

4. A urea as claimed in claim 1 having the formula:

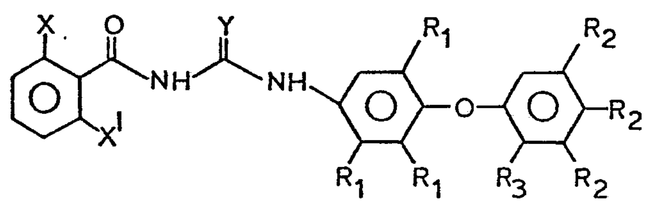

wherein X, X′, Y, $R_1$, $R_2$, $R_3$, m and n are as indicated in claim 1.

5. A urea as claimed in claim 1 having the formula:

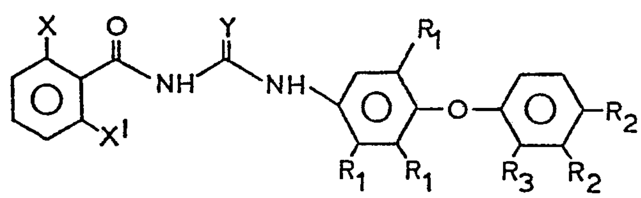

wherein X, X′, Y, $R_1$, $R_2$, $R_3$, m and n are as indicated in claim 1.

6. A urea as claimed in claim 1 having the formula:

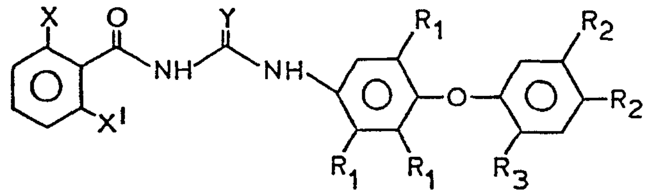

wherein X, X', Y, $R_1$, $R_2$, $R_3$, m and n are as indicated in claim 1.

7. A urea as claimed in claim 1 having the formula:

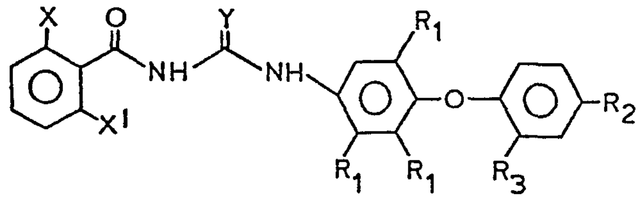

wherein X, X', Y, $R_1$, $R_2$, $R_3$, m and n are as indicated in claim 1.

8. A urea as claimed in claim 1 having the formula:

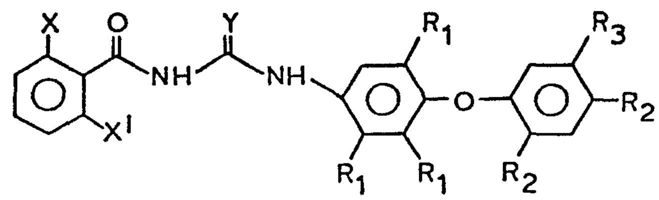

wherein X, X', Y, $R_1$, $R_2$, $R_3$, m and n are as indicated in claim 1.

9. A urea as claimed in any one of the preceding claims wherein Y is oxygen, X is hydrogen or halogen, X' is halogen, $R_1$ is chlorine or methyl, $R_2$ is halogen or $C_1$—$C_4$ alkyl, $R_3$ is $C_1$—$C_4$ alkyl, m is 3 and n is 1.

10. 1-[4-(2,4-dimethylphenoxy)-3,6-dimethyl-5-chlorophenyl]-3-(2-chlorobenzoyl)urea.

11. 1-[4-(2,4-dimethylphenoxy)-3,6-dimethyl-5-chlorophenyl]-3-(2,6-difluorobenzoyl)urea.

12. 1-[4-(3,5-dimethylphenoxy)-3,6-dimethyl-5-chlorophenyl]-(2,6-difluorobenzoyl)urea.

13. 1-[4-(2-methyl-4-bromophenoxy)-3,6-dimethyl-5-chlorophenyl]-3-(2-chlorobenzoyl)urea.

14. 1-[4-(2-methyl-4-bromophenoxy)-3,6-dimethyl-5-chlorophenyl]-3-(2,6-difluorobenzoyl)urea.

15. 1-[4-(2-methyl-4-chlorophenoxy)-3,6-dimethyl-5-chlorophenyl]-3-(2-chlorobenzoyl)urea.

16. 1-[4-(2-methyl-4-chlorophenoxy)-3,6-dimethyl-5-chlorophenyl]-3-(2,6-difluorobenzoyl)urea.

17. 1-[4-(2-methyl-4-tertiary-butylphenoxy)-3,6-dimethyl-5-chlorophenyl]-3-(2,6-difluorobenzoyl)urea.

18. 1-[4-(2,3,5-trimethylphenoxy)-3,6-dimethyl-5-chlorophenyl]-3-(2,6-difluorobenzoyl)urea.

19. 1-[4-(3,5-dimethyl-4-chlorophenoxy)-3,6-dimethyl-5-chlorophenyl]-3-(2,6-difluorobenzoyl)urea.

20. 1-[4-(2,3-dimethyl-4-bromophenoxy)-3,6-dimethyl-5-chlorophenyl]-3-(2-chlorobenzoyl)urea.

21. 1-[4-(2,5-dimethyl-4-chlorophenoxy)-3,6-dimethyl-5-chlorophenyl]-3-(2,6-difluorobenzoyl)urea.

22. 1-[4-(2,5-dimethylphenoxy)-3,6-dimethyl-5-chlorophenyl]-3-(2,6-difluorobenzoyl)urea.

23. 1-[4-(2,5-dimethylphenoxy)-3,6-dimethyl-5-chlorophenyl]-3-(2-chlorobenzoyl)urea.

24. 1-[4-(2,4,5-trimethylphenoxy)-3,6-dimethyl-5-chlorophenyl]-3-(2,6-difluorobenzoyl)urea.

25. 1-[4-(2,4,5-trimethylphenoxy)-3,6-dimethyl-5-chlorophenyl]-3-(2-chlorobenzoyl)urea.

26. 1-[4-(2,5-dimethyl-4-bromophenoxy)-3,6-dimethyl-5-chlorophenyl]-3-(2,6-difluorobenzoyl)urea.

27. 1-[4-(2,5-dimethyl-4-cyanophenoxy)-3,6-dimethyl-5-chlorophenyl]-3-(2,6-difluorobenzoyl)urea.

28. 1-[4-(2,5-dimethyl-4-trifluoromethylphenoxy)-3,6-dimethyl-5-chlorophenyl]-3-(2,6-difluorobenzoyl)urea.

29. A pesticide composition comprising an acceptable carrier and a pesticidally effective amount of a urea as claimed in any one of the preceding claims.

30. A method of controlling pests which comprises subjecting said pests to a pesticidally effective amont of a urea or of a composition as claimed in any of the preceding claims.

31. A method of preparing a 1-(alkylphenoxyaryl)-3-benzoyl urea of the general formula:

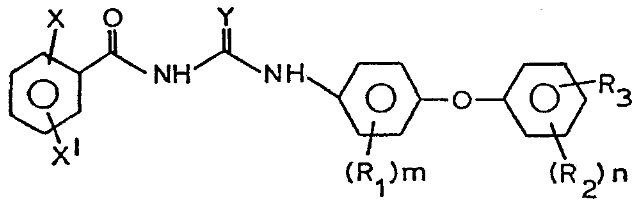

wherein

X, X′ are independently hydrogen, halogen, $C_1$—$C_4$ alkyl, haloalkyl, polyhaloalkyl, alkoxy, polyhaloalkoxy,

Y represents oxygen or sulfur;

m, n are independently 1—4;

$R_1$ represents halogen, $C_1$—$C_4$ alkyl, haloalkyl, polyhaloalkyl, or alkoxy,

$R_2$ represents halogen, $C_1$—$C_4$ alkyl, polyhaloalkyl, polyhaloalkoxy, $C_1$—$C_8$ alkylsulfonyl, $C_1$—$C_8$ alkoxy, $C_1$—$C_8$ alkylthio, $C_1$—$C_8$ dialkylamino, CN, $NO_2$, $CO_2R_4$, or $CONHR_4$ wherein $R_4$ represents $C_1$—$C_8$ alkyl; and

$R_3$ represents $C_1$—$C_{12}$ alkyl, with the proviso that m may not be 0 or 1 when n is less than 2, which method comprises reacting a benzoyl isocyanate or a benzoyl isothiocyanate of the formula:

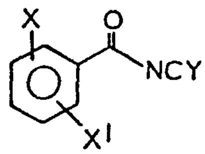

with an alkylphenoxyaniline of the formula:

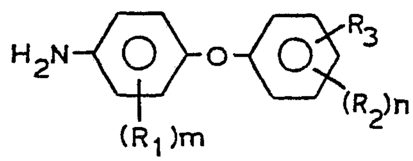

wherein X, X′, Y, $R_1$, $R_2$, $R_3$, m and n have the meanings set forth above, and thereafter recovering the urea.

32. A method of preparing a 1-(alkylphenoxyaryl)-3-benzoyl urea of the general formula:

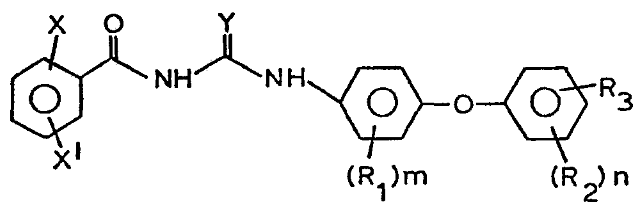

wherein

X, X′ are independently hydrogen, halogen, $C_1$—$C_4$ alkyl, haloalkyl, polyhaloalkyl, alkoxy, polyhaloalkoxy,

Y represents oxygen or sulfur;

m, n are independently 1—4;

$R_1$ represents halogen, $C_1$—$C_4$ alkyl, haloalkyl, polyhaloalkyl, or alkoxy,

$R_2$ represents halogen, $C_1$—$C_4$ alkyl, polyhaloalkyl, polyhaloalkoxy, $C_1$—$C_8$ alkylsulfonyl, $C_1$—$C_8$ alkoxy, $C_1$—$C_8$ alkylthio, $C_1$—$C_8$ dialkylamino, CN, $NO_2$, $CO_2R_4$, or $CONHR_4$ wherein $R_4$ represents $C_1$—$C_8$ alkyl; and

$R_3$ represents $C_1$—$C_{12}$ alkyl, with the proviso that m may not be 0 or 1 when n is less than 2, which method comprises reacting an alkylphenoxyphenyl isocyanate or alkylphenoxyphenyl isothiocyanate of the formula:

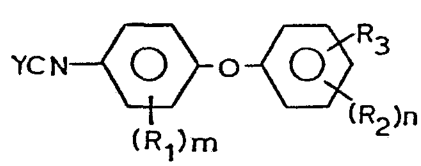

with a benzamide of the formula:

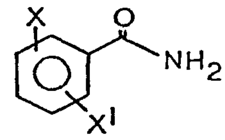

wherein X, X', Y, $R_1$, $R_2$, $R_3$, m and n have the same meanings set forth above, and thereafter recovering the urea.

33. A method as claimed in claim 31 or 32 wherein the urea is 1-[4-(2,4-dimethylphenoxy)-3,6-dimethyl-5-chlorophenyl]-3-(2-chlorobenzoyl)urea.

34. A method as claimed in claim 31 or 32 wherein the urea is 1-[4-(2,4-dimethylphenoxy)-3,6-dimethyl-5-chlorophenyl]-3-(2,6-difluorobenzoyl)urea.

**Patentansprüche**

1. 1-(Alkylphenoxyaryl)-3-benzoylharnstoff der allgemeinen Formel:

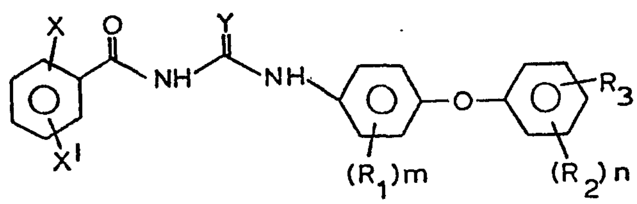

worin

X, X' unabhängig voneinander Wasserstoff, Halogen, $C_1$—$C_4$-Alkyl, Halogenalkyl, Polyhalogenalkyl, Alkoxy, Polyhalogenalkoxy sind;

Y für Sauerstoff oder Schwefel steht;

m, n unabhängig voneinander 1 bis 4 sind;

$R_1$ für Halogen, $C_1$—$C_4$-Alkyl, Halogenalkyl, Polyhalogenalkyl oder Alkoxy steht;

$R_2$ für Halogen, $C_1$—$C_4$-Alkyl, Polyhalogenalkyl, Polyhalogenalkoxy, $C_1$—$C_8$-Alkylsulfonyl, $C_1$—$C_8$-Alkoxy, $C_1$—$C_8$-Alkylthio, $C_1$—$C_8$-Dialkylamino, CN, $NO_2$, $CO_2R_4$ oder $CONHR_4$ steht, worin $R_4$ für $C_1$—$C_8$-Alkyl steht; und

$R_3$ für $C_1$—$C_{12}$-Alkyl steht, unter der Voraussetzung, daß m weder 0 noch 1 ist, wenn n kleiner als 2 ist.

2. Harnstoffderivat nach Anspruch 1 der Formel:

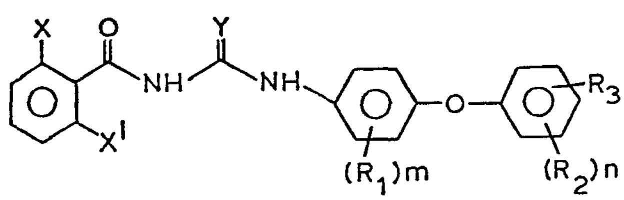

worin X, X', Y, $R_1$, $R_2$, $R_3$, m und n dieselbe Bedeutung wie in Anspruch 1 haben.

3. Harnstoffderivat nach Anspruch 1 der Formel:

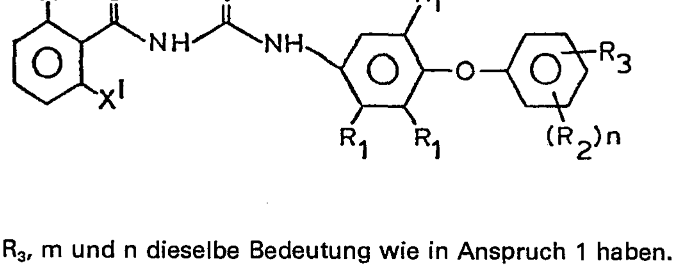

worin X, X', Y, $R_1$, $R_2$, $R_3$, m und n dieselbe Bedeutung wie in Anspruch 1 haben.

4. Harnstoffderivat nach Anspruch 1 der Formel:

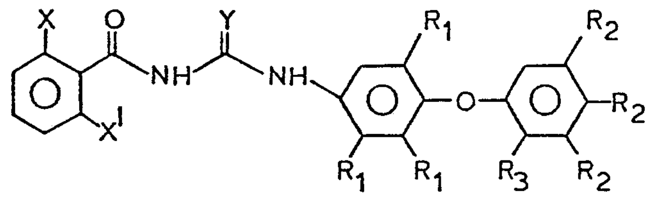

worin X, X', Y, $R_1$, $R_2$, $R_3$, m und n dieselbe Bedeutung wie in Anspruch 1 haben.

5. Harnstoffderivat nach Anspruch 1 der Formel:

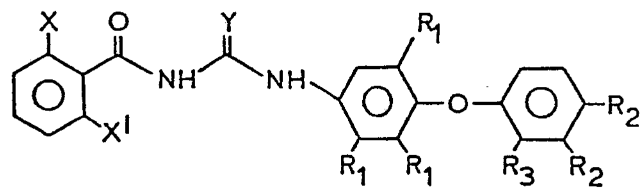

worin X, X', Y, $R_1$, $R_2$, $R_3$, m und n dieselbe Bedeutung wie in Anspruch 1 haben.

6. Harnstoffderivat nach Anspruch 1 der Formel:

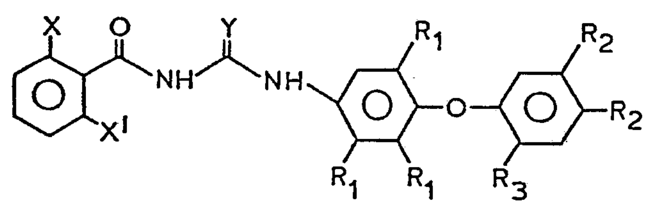

worin X, X', Y, $R_1$, $R_2$, $R_3$, m und n dieselbe Bedeutung wie in Anspruch 1 haben.

7. Harnstoffderivat nach Anspruch 1 der Formel:

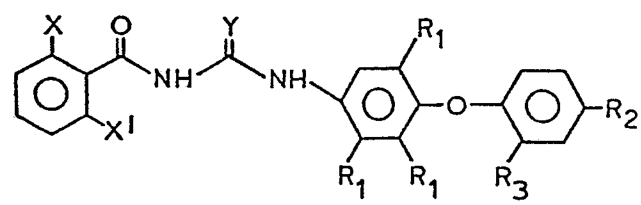

worin X, X', Y, $R_1$, $R_2$, $R_3$, m und n dieselbe Bedeutung wie in Anspruch 1 haben.

8. Harnstoffderivat nach Anspruch 1 der Formel:

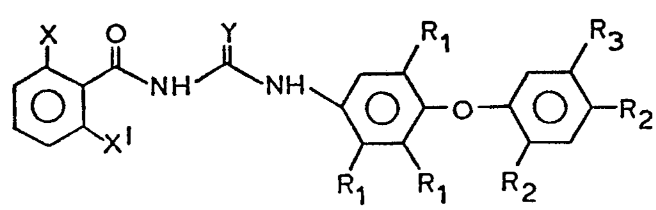

worin X, X', Y, $R_1$, $R_2$, $R_3$, m und n dieselbe Bedeutung wie in Anspruch 1 haben.

9. Harnstoffderivat nach irgendeinem der vorangehenden Ansprüche, worin Y Sauerstoff, X Wasserstoff oder Halogen, X' Halogen, $R_1$ Chlor oder Methyl, $R_2$ Halogen oder $C_1$—$C_4$-Alkyl, $R_3$ $C_1$—$C_4$-Alkyl, m = 3 und n = 1 ist.

10. 1-[4-(2,4-Dimethylphenoxy)-3,6-dimethyl-5-chlorphenyl]-3-(2-chlorbenzoyl)harnstoff.

11. 1-[4-(2,4-Dimethylphenoxy)-3,6-dimethyl-5-chlorphenyl]-3-(2,6-difluorbenzoyl)harnstoff.

12. 1-[4-(3,5-Dimethylphenoxy)-3,6-dimethyl-5-chlorphenyl]-(2,6-difluorbenzoyl)harnstoff.

13. 1-[4-(2-Methyl-4-bromphenoxy)-3,6-dimethyl-5-chlorphenyl]-3-(2-chlorbenzoyl)harnstoff.

14. 1-[4-(2-Methyl-4-bromphenoxy)-3,6-dimethyl-5-chlorphenyl]-3-(2,6-difluorbenzoyl)harnstoff.

15. 1-[4-(2-Methyl-4-chlorphenoxy)-3,6-dimethyl-5-chlorphenyl]-3-(2-chlorbenzoyl)harnstoff.
16. 1-[4-(2-Methyl-4-chlorphenoxy)-3,6-dimethyl-5-chlorphenyl)-3-(2,6-difluorbenzoyl)harnstoff.
17. 1-[4-(2-Methyl-4-tertiär-butylphenoxy)-3,6-dimethyl-5-chlorphenyl]-3-(2,6-difluorbenzoyl)harnstoff.
18. 1-[4-(2,3,5-Trimethylphenoxy)-3,6-dimethyl-5-chlorphenyl]-3-(2,6-difluorbenzoyl)harnstoff.
19. 1-[4-(3,5-Dimethyl-4-chorphenoxy)-3,6-dimethyl-5-chlorphenyl]-3-(2,6-difluorbenzoyl)harnstoff.
20. 1-[4-(2,3-Dimethyl-4-bromphenoxy)-3,6-dimethyl-5-chlorphenyl]-3-(2-chlorbenzoyl)harnstoff.
21. 1-[4-(2,5-dimethyl-4-chlorphenoxy)-3,6-dimethyl-5-chlorphenyl]-3-(2,6-difluorbenzoyl)harnstoff.
22. 1-[4-(2,5-Dimethylphenoxy)-3,6-dimethyl-5-chlorphenyl]-3-(2,6-difluorbenzoyl)harnstoff.
23. 1-[4-(2,5-Dimethylphenoxy)-3,6-dimethyl-5-chlorphenyl]-3-(2-chlorbenzoyl)harnstoff.
24. 1-[4-(2,4,5-Trimethylphenoxy)-3,6-dimethyl-5-chlorphenyl]-3-(2,6-difluorbenzoyl)harnstoff.
25. 1-[4-(2,4,5-Trimethylphenoxy)-3,6-dimethyl-5-chlorphenyl]-3-(2-chlorbenzoyl)harnstoff.
26. 1-[4-(2,5-Dimethyl-4-bromphenoxy)-3,6-dimethyl-5-chlorphenyl]-3-(2,6-difluorbenzoyl)harnstoff.
27. 1-[4-(2,5-Dimethyl-4-cyanophenoxy)-3,6-dimethyl-5-chlorphenyl]-3-(2,6-difluorbenzoyl)harnstoff.
28. 1-[4-(2,5-Dimethyl-4-trifluormethylphenoxy)-3,6-dimethyl-5-chlorphenyl]-3-(2,6-difluorbenzoyl)harnstoff.

29. Pestizide Zusammensetzung, die einen geeigneten Träger und eine pestizidisch wirksame Menge eines Harnstoffderivats nach irgendeinem der vorangehenden Ansprüche umfaßt.

30. Verfahren zur Schädlingsbekämpfung, das die Behandlung der Schädlinge mit einer pestizidisch wirksamen Menge eines Harnstoffderivats oder einer Zusammensetzung nach irgendeinem der vorangehenden Ansprüche umfaßt.

31. Verfahren zur Herstellung von 1-(Alkylphenoxyaryl)-3-benzoylharnstoff der allgemeinen Formel:

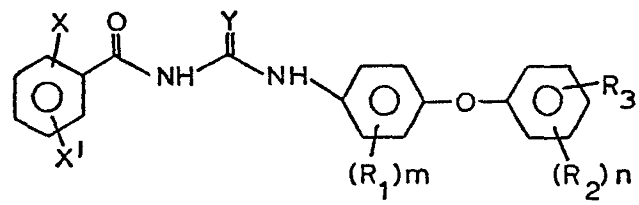

worin:

X, X′ unabhängig voneinander Wasserstoff, Halogen, $C_1$—$C_4$-Alkyl, Halogenalkyl, Polyhalogenalkyl, Alkoxy, Polyhalogenalkoxy sind;

Y für Sauerstoff oder Schwefel steht;

m, n unabhängig voneinander 1 bis 4 sind;

$R_1$ für Halogen, $C_1$—$C_4$-Alkyl, Halogenalkyl, Polyhalogenalkyl oder Alkoxy steht;

$R_2$ für Halogen, $C_1$—$C_4$-Alkyl, Polyhalogenalkyl, Polyhalogenalkoxy, $C_1$—$C_8$-Alkylsulfonyl, $C_1$—$C_8$-Alkoxy, $C_1$—$C_8$-Alkylthio, $C_1$—$C_8$-Dialkylamino, CN, $NO_2$, $CO_2R_4$ oder $CONHR_4$ steht, worin $R_4$ für $C_1$—$C_8$-Alkyl steht; und

$R_3$ für $C_1$—$C_{12}$-Alkyl steht, unter der Voraussetzung, daß m weder 0 noch 1 ist, wenn n kleiner als 2 ist, wobei das Verfahren die Reaktion eines Benzoylisocyanats oder eines Benzoylisothiocyanats der Formel:

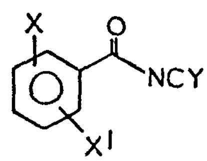

mit einem Alkylphenoxyanilin der Formel:

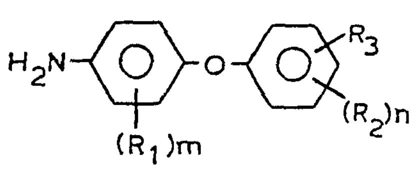

worin X, X′, Y, $R_1$, $R_2$, $R_3$, m und n die oben genannten Bedeutungen haben, und danach die Gewinnung des Harnstoffderivats umfaßt.

32. Verfahren zur Herstellung von 1-(Alkylphenoxyaryl)-3-benzoylharnstoff der allgemeinen Formel:

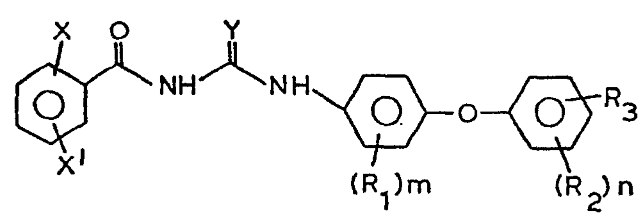

worin

X, X' unabhängig voneinander Wasserstoff, Halogen, $C_1$—$C_4$-Alkyl, Halogenalkyl, Polyhalogenalkyl, Alkoxy, Polyhalogenalkoxy sind;

Y für Sauerstoff oder Schwefel steht;

m, n unabhängig voneinander 1 bis 4 sind;

$R_1$ für Halogen, $C_1$—$C_4$-Alkyl, Halogenalkyl, Polyhalogenalkyl oder Alkoxy steht;

$R_2$ für Halogen, $C_1$—$C_4$-Alkyl, Polyhalogenalkyl, Polyhalogenalkoxy, $C_1$—$C_8$-Alkylsulfonyl, $C_1$—$C_8$-Alkoxy, $C_1$—$C_8$-Alkylthio, $C_1$—$C_8$-Dialkylamino, CN, $NO_2$, $CO_2R_4$ oder $CONHR_4$ steht, worin $R_4$ für $C_1$—$C_8$-Alkyl steht; und

$R_3$ für $C_1$—$C_{12}$-Alkyl steht, unter der Voraussetzung, daß m weder 0 noch 1 ist, wenn n kleiner als 2 ist, wobei das Verfahren die Reaktion eines Alkylphenoxyphenylisocyanats oder Alkylphenoxyphenylisothiocyanats der Formel:

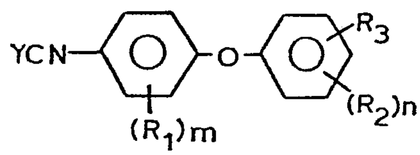

mit einem Benzamid der Formel:

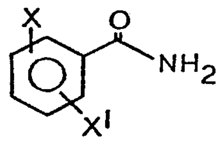

worin X, X', Y, $R_1$, $R_2$, $R_3$, m und n die oben genannte Bedeutung haben, und danach die Gewinnung des Harnstoffderivats umfaßt.

33. Verfahren nach Anspruch 31 oder 32, worin das Harnstoffderivat 1-[4-(2,4-Dimethylphenoxy)-3,6-dimethyl-5-chlorphenyl]-3-(2-chlorbenzoyl)harnstoff ist.

34. Verfahren nach Anspruch 31 oder 32, worin das Harnstoffderivat 1-[4-(2,4-Dimethylphenoxy)-3,6-dimethyl-5-chlorphenyl]-3-(2,6-difluorbenzoyl)harnstoff ist.

**Revendications**

1. 1-(alkyl-phénoxyaryl)-3-benzoyl-urée de formule générale;

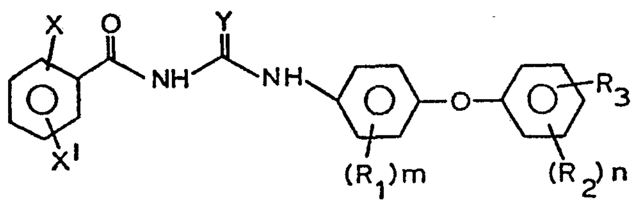

dans laquelle:

X, X' représentent indépendamment l'hydrogène, un halogène, un groupe alkyle, halogénalkyle, polyhalogénalkyle, alkoxy, polyhalogénalkoxy en $C_1$ à $C_4$;

Y représente l'oxygène ou le soufre;

m, n ont, indépendamment, des valeurs de 1 à 4;

$R_1$ représente un halogène, un groupe alkyle, halogénalkyle, polyhalogénalkyle ou alkoxy en $C_1$ à $C_4$;

$R_2$ représente un halogène, un groupe alkyle, polyhalogénalkyle, polyhalogénalkoxy en $C_1$ à $C_4$, alkylsulfonyle en $C_1$ à $C_8$, alkoxy en $C_1$ à $C_8$, alkylthio en $C_1$ à $C_8$, di(alkyle en $C_1$ à $C_8$)amino, CN, $NO_2$, $CO_2R_4$ ou $CONHR_4$ où $R_4$ représente un radical alkyle en $C_1$ à $C_8$; et

$R_3$ représente un groupe alkyle en $C_1$ à $C_{12}$, sous réserve que m ne puisse pas être égal à 0 ou à 1 lorsque n est inférieur à 2.

2. Urée suivant la revendication 1, répondant à la formule:

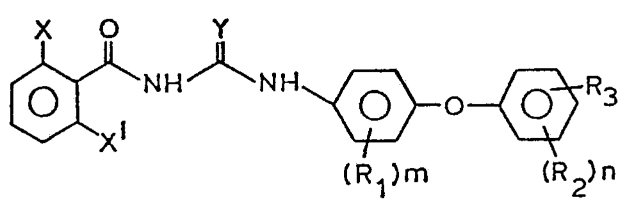

dans laquelle X, X' Y, $R_1$, $R_2$, $R_3$, m et n sont tels que définis dans la revendication 1.

3. Urée suivant la revendication 1, répondant à la formule:

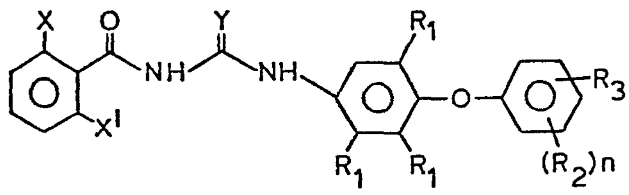

dans laquelle X, X' Y, $R_1$, $R_2$, $R_3$, m et n sont tels que définis dans la revendication 1.

4. Urée suivant la revendication 1, répondant à la formule:

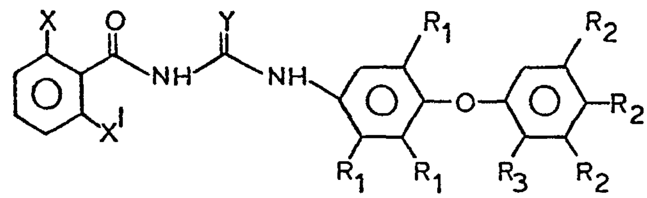

dans laquelle X, X' Y, $R_1$, $R_2$, $R_3$, m et n sont tels que définis dans la revendication 1.

5. Urée suivant la revendication 1, répondant à la formule:

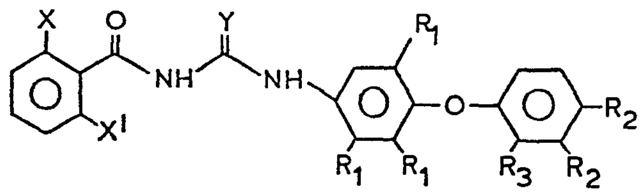

dans laquelle X, X' Y, $R_1$, $R_2$, $R_3$, m et n sont tels que définis dans la revendication 1.

6. Urée suivant la revendication 1, répondant à la formule:

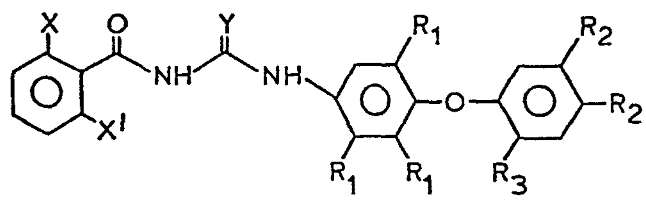

dans laquelle X, X' Y, $R_1$, $R_2$, $R_3$, m et n sont tels que définis dans la revendication 1.

7. Urée suivant la revendication 1, répondant à la formule:

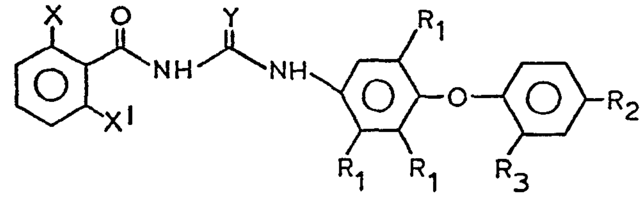

dans laquelle X, X' Y, $R_1$, $R_2$, $R_3$, m et n sont tels que définis dans la revendication 1.

8. Urée suivant la revendication 1, répondant à la formule:

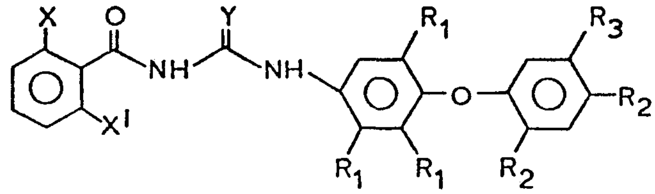

dans laquelle X, X' Y, $R_1$, $R_2$, $R_3$, m et n sont tels que définis dans la revendication 1.

9. Urée suivant l'une quelconque des revendications précédentes, dans laquelle Y est l'oxygène, X est l'hydrogène ou un halogène, X' est un halogène, $R_1$ est le chlore ou le groupe méthyle, $R_2$ est un halogène ou un groupe alkyle en $C_1$ à $C_4$, $R_3$ est un groupe alkyle en $C_1$ à $C_4$, m est égal à 3 et n est égal à 1.

10. La 1-[4-(2,4-diméthylphénoxy)-3,6-diméthyl-5-chlorophényl]-3-(2-chlorobenzoyl)-urée.

11. La 1-[4-(2,4-diméthylphénoxy)-3,6-diméthyl-5-chlorophényl]-3-(2,6-difluorobenzoyl)-urée.

12. La 1-[4-(3,5-diméthylphénoxy)-3,6-diméthyl-5-chlorophényl]-(2,6-difluorobenzoyl)-urée.

13. La 1-[4-(2-méthyl-4-bromophénoxy)-3,6-diméthyl-5-chlorophényl]-3-(2-chlorobenzoyl)-urée.

14. La 1-[4-(2-méthyl-4-bromophénoxy)-3,6-diméthyl-5-chlorophényl]-3-(2,6-difluorobenzoyl)-urée.

15. La 1-[4-(2-méthyl-4-chlorophénoxy)-3,6-diméthyl-5-chlorophényl]-3-(2-chlorobenzoyl)-urée.

16. La 1-[4-(2-méthyl-4-chlorophénoxy)-3,6-diméthyl-5-chlorophényl]-3-(2,6-difluorobenzoyl)-urée.

17. La 1-[4-(2-méthyl-4-tertio-butylphénoxy)-3,6-diméthyl-5-chlorophényl]-3-(2,6-difluorobenzoyl)-urée.

18. La 1-[4-(2,3,5-triméthylphénoxy)-3,6-diméthyl-5-chlorophényl]-3-(2,6-difluorobenzoyl)-urée.

19. La 1-[4-(3,5-diméthyl-4-chlorophénoxy)-3,6-diméthyl-5-chlorophényl]-3-(2,6-difluorobenzoyl)-urée.

20. La 1-[4-(2,3-diméthyl-4-bromophénoxy)-3,6-diméthyl-5-chlorophényl]-3-(2-chlorobenzoyl)-urée.

21. La 1-[4-(2,5-diméthyl-4-chlorophénoxy)-3,6-diméthyl-5-chlorophényl]-3-(2,6-difluorobenzoyl)-urée.

22. La 1-[4-(2,5-diméthylphénoxy)-3,6-diméthyl-5-chlorophényl]-3-(2,6-difluorobenzoyl)-urée.

23. La 1-[4-(2,5-diméthylphénoxy)-3,6-diméthyl-5-chlorophényl]-3-(2-chlorobenzoyl)-urée.

24. La 1-[4-(2,4,5-triméthylphénoxy)-3,6-diméthyl-5-chlorophényl]-3-(2,6-difluorobenzoyl)-urée.

25. La 1-[4-(2,4,5-triméthylphénoxy)-3,6-diméthyl-5-chlorophényl]-3-(2-chlorobenzoyl)-urée.

26. La 1-[4-(2,5-diméthyl-4-bromophénoxy)-3,6-diméthyl-5-chlorophényl]-3-(2,6-difluorobenzoyl)-urée.

27. La 1-[4-(2,5-diméthyl-4-cyanophénoxy)-3,6-diméthyl-5-chlorophényl]-3-(2,6-difluorobenzoyl)-urée.

28. La 1-[4-(2,5-diméthyl-4-trifluorométhylphénoxy)-3,6-diméthyl-5-chlorophényl]-3-(2,6-difluorobenzoyl)-urée.

29. Composition pesticide comprenant un support acceptable et une quantité efficace du point de vue pesticide d'une urée suivant l'une quelconque des revendications précédentes.

30. Procédé pour combattre des parasites, qui consiste à soumettre lesdits parasites à une quantité efficace du point du vue pesticide d'une urée ou d'une composition suivant l'une quelconque des revendications précédentes.

31. Procédé de préparation d'une 1-(alkylphénoxyaryl)-3-benzoyl-urée de formule générale:

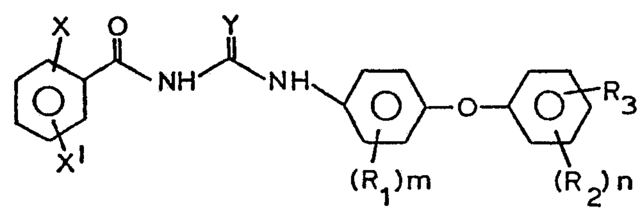

dans laquelle:

X, X' représentent indépendamment l'hydrogène, un halogène, un groupe alkyle, halogénalkyle, polyhalogénalkyle, alkoxy, polyhalogénalkoxy en $C_1$ à $C_4$;

Y représente l'oxygène ou le soufre;

m, n ont, indépendamment, des valeurs de 1 à 4;

$R_1$ représente un halogène, un groupe alkyle, halogénalkyle, polyhalogénalkyle ou alkoxy en $C_1$ à $C_4$;

$R_2$ représente un halogène, un groupe alkyle, polyhalogénalkyle, polyhalogénalkoxy en $C_1$ à $C_4$, alkylsulfonyle en $C_1$ à $C_8$, alkoxy en $C_1$ à $C_8$, alkylthio en $C_1$ à $C_8$, di(alkyle en $C_1$ à $C_8$)amino, CN, $NO_2$, $CO_2R_4$ ou $CONHR_4$ où $R_4$ représente un radical alkyle en $C_1$ à $C_8$; et

$R_3$ représente un groupe alkyle en $C_1$ à $C_{12}$, sous réserve que m ne puisse pas être égal à 0 ou à 1 lorsque n est inférieur à 2, procédé qui consiste à faire réagir un isocyanate de benzoyle ou un isothiocyanate de benzoyle de formule:

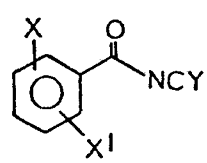

avec une alkylphénoxyaniline de formule:

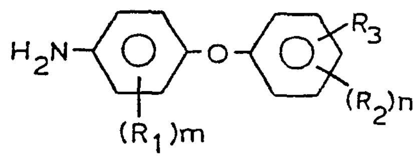

dans laquelle X, X' Y, $R_1$, $R_2$, $R_3$, m et n ont les définitions indiquées ci-dessus, puis à recueillir l'urée.

32. Procédé de préparation d'une 1-(alkylphénoxyaryl)-3-benzoyl-urée de formule générale:

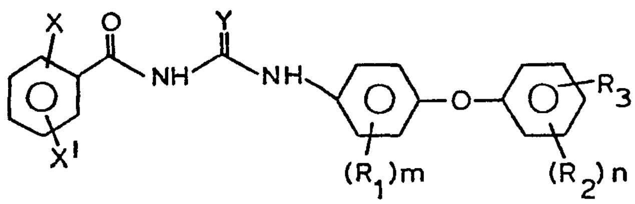

dans laquelle:

X, X' représentent indépendamment l'hydrogène, un halogène, un groupe alkyle, halogénalkyle, polyhalogénalkyle, alkoxy, polyhalogénalkoxy en $C_1$ à $C_4$;

Y représente l'oxygène ou le soufre;

m, n ont, indépendamment, des valeurs de 1 à 4;

$R_1$ représente un halogène, un groupe alkyle, halogénalkyle, polyhalogénalkyle ou alkoxy en $C_1$ à $C_4$;

$R_2$ représente un halogène, un groupe alkyle, polyhalogénalkyle, polyhalogénalkoxy en $C_1$ à $C_4$, alkylsulfonyle en $C_1$ à $C_8$, alkoxy en $C_1$ à $C_8$, alkylthio en $C_1$ à $C_8$, di(alkyle en $C_1$ à $C_8$)amino, CN, $NO_2$, $CO_2R_4$ ou $CONHR_4$ où $R_4$ représente un radical alkyle en $C_1$ à $C_8$; et

$R_3$ représente un groupe alkyle en $C_1$ à $C_{12}$, sous réserve que m ne puisse pas être égal à 0 ou à 1 lorsque n est inférieur à 2, procédé qui consiste à faire réagir un isocyanate d'alkylphénoxyphényle ou un isothiocyanate d'alkylphénoxyphényle de formule:

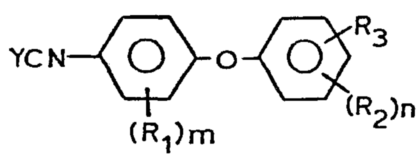

avec une benzamide de formule:

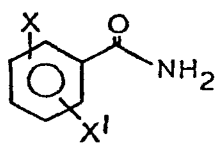

dans laquelle X, X' Y, $R_1$, $R_2$, $R_3$, m et n ont les définitions indiquées ci-dessus, puis à recueillir l'urée.

33. Procédé suivant la revendication 31 ou 32, dans lequel l'urée est la 1-[4-(2,4-diméthylphénoxy)-3,6-diméthyl-5-chlorophényl]-3-(2-chlorobénzoyl)-urée.

34. Procédé suivant la revendication 31 ou 32, dans lequel l'urée est la 1-[4-(2,4-diméthylphénoxy)-3,6-diméthyl-5-chlorophényl]-3-(2,6-difluorobenzoyl)-urée.